# EUROPÄISCHE PATENTSCHRIFT

(11) **EP 1 194 633 B1**
(45) Veröffentlichungstag und Bekanntmachung des Hinweises auf die Patenterteilung: **30.04.2008**
(21) Anmeldenummer: 01919377.0
(22) Anmeldetag: 09.03.2001
(51) Int. Cl.: A61Q 5/06, A61K 8/41, A61K 8/43, A61K 8/49, D06P 5/13

(54) **MITTEL ZUR FÄRBUNG VON FASERN UND VERFAHREN ZUM TEMPORÄREN FÄRBEN VON FASERN**
AGENT FOR COLORING FIBERS AND METHOD FOR TEMPORARILY COLORING FIBERS
AGENT PERMETTANT DE COLORER DES FIBRES ET PROCEDE DE COLORATION TEMPORAIRE DE FIBRES

(30) Priorität: 10.05.2000 DE 10022744
(43) Veröffentlichungstag der Anmeldung: 10.04.2002
(73) Patentinhaber: Wella Aktiengesellschaft, 64274 Darmstadt (DE)
(72) Erfinder: SAUTER, Guido, CH-1723 Marly (CH); BRAUN, Hans-Jürgen, CH-3182 Überstorf (CH); REICHLIN, Nadia, CH-1482 Cugy (CH)
(86) Internationale Anmeldenummer: PCT/EP2001/002685
(87) Internationale Veröffentlichungsnummer: WO 2001/086057

(56) Entgegenhaltungen:
- EP-A- 0 270 972
- GB-A- 1 528 590

## Beschreibung

Gegenstand der vorliegenden Erfindung ist ein Mittel zur Färbung von Fasern, insbesondere von menschlichen Haaren, welches Enamine und Schiffsche Basen enthält, wobei -falls gewünscht- die erhaltene Färbung zu einem beliebigen späteren Zeitpunkt auch wieder schonend entfärbt werden kann.

Haarfärbemittel werden je nach zu färbender Ausgangshaarfarbe und gewünschtem Endresultat hauptsächlich in den Bereich der Oxidationsfärbemittel oder der Tönungen unterteilt. Oxidationshaarfarben eignen sich hervorragend für die Abdeckung von höheren Grauanteilen, hierbei werden die bei einem Grauanteil von bis zu 50 % verwendeten Oxidationsfärbemittel in der Regel als oxidative Tönungen bezeichnet, während die bei einem Grauanteil von über 50 % oder zum "Hellerfärben" verwendeten Oxidationsfärbemittel in der Regel als sogenannte oxidative Farben bezeichnet werden. Direktziehende Farbstoffe sind hauptsächlich in nicht-oxidativen Färbemitteln (sogenannten Tönungsmitteln) enthalten. Einige direktziehende Farbstoffe wie z. B. die Nitrofarbstoffe, können aufgrund ihrer geringen Größe in das Haar eindringen und es - zumindestens in den äußeren Bereichen - direkt anfärben. Derartige Tönungen sind sehr haarschonend und überstehen in der Regel 6 bis 8 Haarwäschen und ermöglichen eine Grauabdeckung von etwa 20 %.

Im allgemeinen waschen sich direktziehende und oxidative Tönungen nach einigen Haarwäschen heraus. Die Zeitdauer hängt unter anderem sehr stark von der Haarstruktur und der verwendeten Nuance ab. Oxidative Farben können teilweise mit der Zeit verblassen, verbleiben aber in der Regel bis zum nächsten Haarschnitt im Haar. Eine jederzeit mögliche Entfernung der Haarfärbung kann jedoch dann wünschenswert sein, wenn man eine besondere Farbe nur für einen bestimmten Zeitraum tragen will, oder eine Färbung dem Anwender nicht gefällt. Ebenso kann im Falle der Haarfärbung bei Erstverwendern die Möglichkeit einer schonenden und vollständigen Entfernung der Färbung die Angst vor einer zu drastischen Farbveränderung vermindern ("Färbung auf Probe").

Aus der DE-OS 197 17 280 sowie der DE-OS 197 17 281 ist die Verwendung einer Kombination von bestimmten heterozyklischen Carbonylverbindungen beziehungsweise Benzylidenketonen mit Aminen und/oder Hydroxyverbindungen und/oder CH-aciden Verbindungen zur Färbung von Haaren ohne Zusatz von Oxidationsmitteln bekannt. Ebenfalls ist es aus der DE-GM 299 08 464 bekannt, dass Haare durch eine Kombination von bestimmten 1,2,3,3-Tetramethyl-3H-indolium-salzen und Carbonylverbindungen auch ohne den Zusatz von Oxidationsmitteln dauerhaft gefärbt werden können.

Es besteht jedoch weiterhin ein großer Bedarf für Färbemittel, die unter milden Bedingungen sowohl intensive als auch schonende Färbungen ermöglichen und -falls-gewünscht- zu einem beliebigen späteren Zeitpunkt wieder entfärbt werden können.

Aufgabe der vorliegenden Erfindung ist es daher, ein Färbemittel zur Verfügung zu stellen, das eine sehr hohe Lagerstabilität aufweist und auch ohne Zusatz von Oxidationsmitteln (wie zum Beispiel Wasserstoffperoxid) zum einen eine schonende, intensive und beständige Färbung der Fasern im Gelb-, Braun-, Grün und Violettbereich mit guten Echtheitseigenschaften (Waschechtheit, Reibechtheit etc.) und zum anderen eine schonende und vollständige Entfernung dieser Färbung zu jedem beliebigen Zeitpunkt ermöglicht.

Überraschenderweise wurde nunmehr gefunden, dass diese Aufgabe durch die Anwendung eines Färbemittels gelöst werden kann, das durch Vermischen zweier Komponenten erhalten wird, wobei die erste Komponente mindestens ein Enamin der Formel (I) oder dessen Säureadditionssalz der Formel (Ia) enthält und die zweite Komponente mindestens eine Schiffsche Base der Formel (II) enthält.

Gegenstand der vorliegenden Erfindung ist daher ein Mittel zur Färbung von Fasern (A), wie zum Beispiel Wolle, Seide, Baumwolle oder Haaren und insbesondere menschlichen Haaren, welches durch Vermischen zweier Komponenten (A1) und (A2) hergestellt wird und dadurch gekennzeichnet ist, dass die Komponente (A1) einen sauren pH-Wert aufweist und mindestens ein Enamin der Formel (III) bis (X) oder dessen Säureadditionssalz enthält,
in denen **X** gleich einem mit zwei C1- bis C4-Alkylgruppen -welche gleich oder verschieden sein können (insbesondere 2 Methylgruppen)-substituierten C-Atom, einem mit einer C1- bis C4-Alkylgruppe und einer Hydroxygruppe substituierten C-Atom, einem Schwefelatom, einem alkylierten Stickstoffatom, einem nichtalkylierten Stickstoffatom oder einem Sauerstoffatom ist; und **R2** gleich einer geradkettigen oder verzweigten C1- bis C8-Alkylgruppe, einer geradkettigen oder verzweigten C1- bis C8-Hydroxyalkylgruppe, oder einer C1- bis C8-Alkoxyalkylgruppe ist, wobei jeweils zwischen den C-Atomen der Alkylkette Sauerstoffatome sitzen können; **R4** gleich Wasserstoff, einer geradkettigen C1- bis C4-Alkylgruppe oder einer verzweigten C1- bis C4-Alkylgruppe ist;
**R5, R6, R7** und **R8** unabhängig voneinander gleich Wasserstoff, einer geradkettigen oder verzweigten C1- bis C4-Alkylgruppe, einer geradkettigen oder verzweigten C1- bis C4-Hydroxyalkylgruppe, einer Hydroxygruppe, einer Methoxygruppe, einer Aminogruppe, einer Monoalkylaminogruppe, einer Dialkylaminogruppe, einer Benzylgruppe oder einem Halogenatom (F, Cl, Br, J) sind; und **A⁻** gleich Chlorid, Bromid, Jodid, Sulfat, Hydrogensulfat, Toluolsulfonat, Benzolsulfonat, Monomethylsulfat, Hexafluorphosphat, Hexafluorantimonat, Tetrafluorborat, Tetraphenylborat, Formiat, Acetat oder Propionat ist, wobei das Chloridion, das Tetrafluoroboration, das Acetation und das Hydrogensulfation besonders bevorzugt sind; 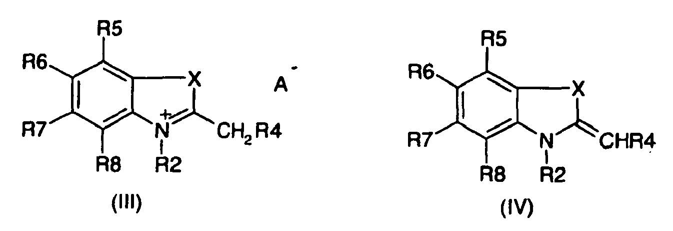 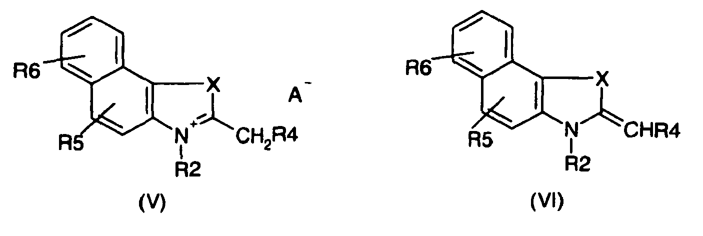 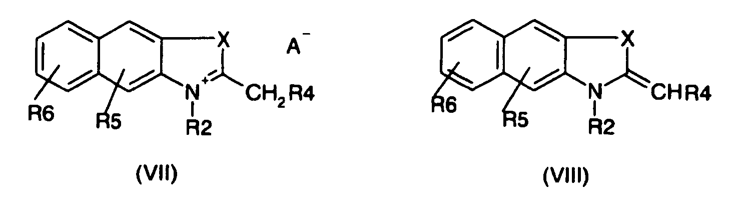 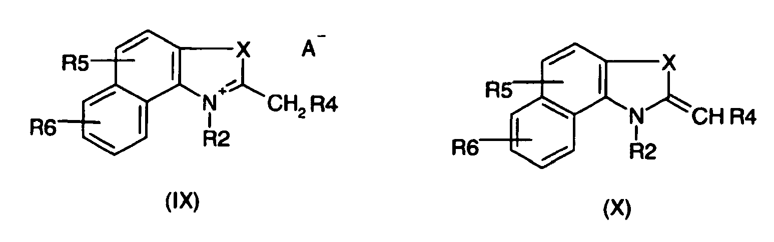 und die Komponente (A2) einen alkalischen pH-Wert aufweist und mindestens eine Schiffsche Base der Formel (II) enthält,

R₇HC=NR₈ (II)

wobei gilt: **R7** ist gleich einer Restgruppe der Formeln 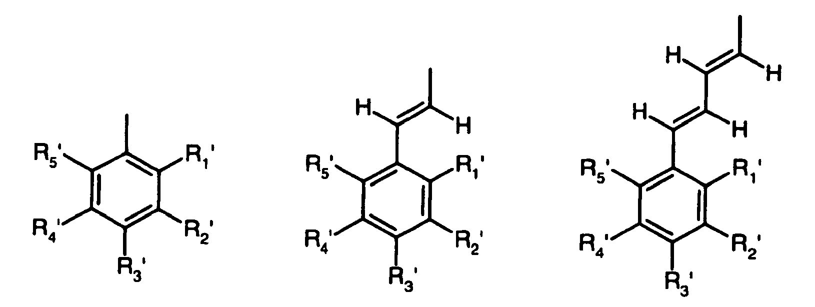 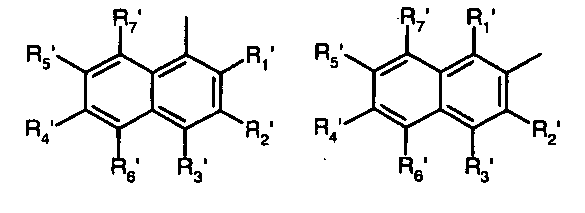 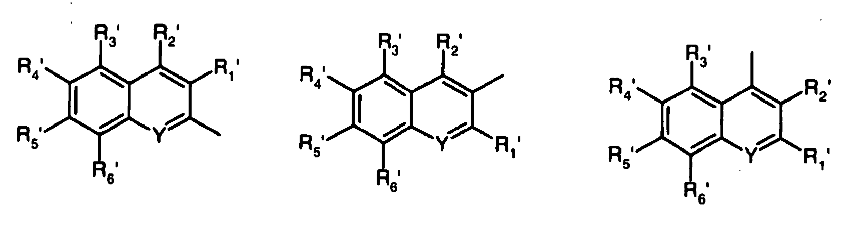 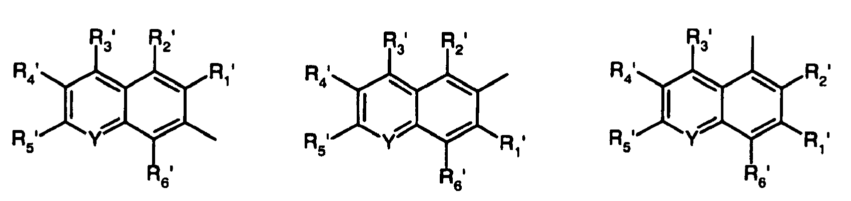 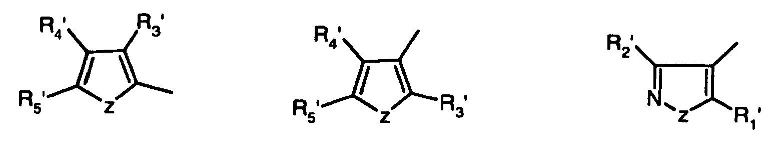 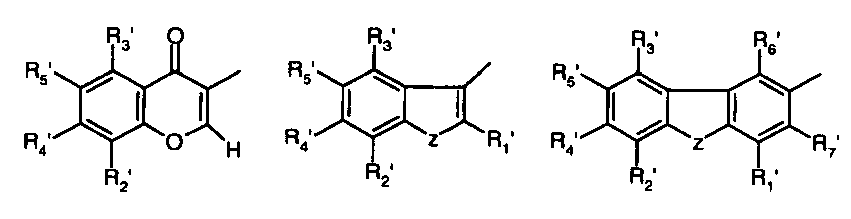 wobei **-Y=** gleich -N=, -N⁺R^{a}=, -O⁺= oder -S⁺= ist, und **Z** gleich einem Sauerstoffatom, einem Schwefelatom oder einer NR^{a}-Gruppe ist; **R1', R2', R3', R4', R5', R6'** und **R7'** unabhängig voneinander gleich einem Wasserstoffatom, einer Hydroxygruppe, einer Methoxygruppe, einer Arylgruppe, einem Halogenatom (F, Cl, Br, J), einer -CHO-Gruppe, einer -COR^{a} -Gruppe, einer -CO₂R^{a} -Gruppe, einer NO₂-Gruppe, einer -OCOR^{a} -Gruppe, einer -OCH₂Aryl-Gruppe, einer -NH₂ -Gruppe, einer -NH₃⁺-Gruppe, einer -NHR^{a}-Gruppe, einer -NR^{a}H₂⁺-Gruppe, einer-N(R^{a})₂-Gruppe, einer -N(R^{a})₃⁺-Gruppe, einer -NHCOR^{a}-Gruppe, einer-NHCOOR^{a}-Gruppe, mit R^{a} gleich einem Wasserstoffatom, einer geradkettigen oder verzweigten C1 bis C4-Alkylgruppe, einem gegebenenfalls substituierten aromatischen Carbozyklus oder Heterocyclus, sind; unter der Bedingung, dass mindestens einer der Reste R1' bis R7' von Wasserstoff verschieden ist; und **R8** gleich einer Restgruppe der Formeln, 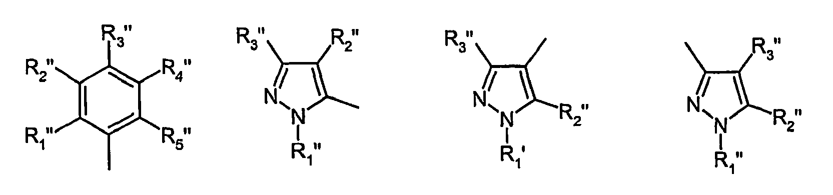 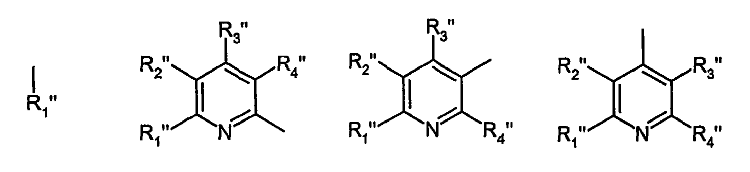 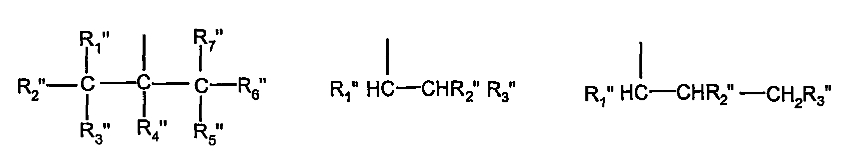 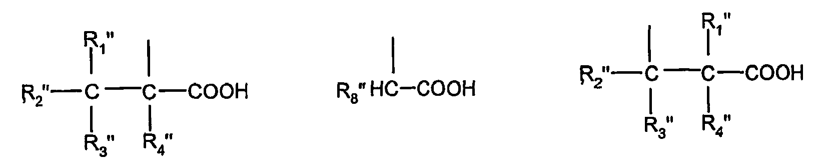 wobei **R1", R2", R3", R4", R5", R6"** und **R7"** unabhängig voneinander gleich einem Wasserstoffatom, einer Methylgruppe, einem Halogenatom, einer Hydroxygruppe, einer C1- bis C4- Hydroxyalkylgruppe, einer Benzylgruppe, einem gegebenenfalls substituierten aromatischen Carbozyklus oder Heterocyclus, einer Methoxygruppe, einer Ethoxy-gruppe, einer Carboxygruppe, einer -NH₂-Gruppe, einer -NHR^{b}-Gruppe, einer-N(R^{b})₂-Gruppe sind, mit R^{b} gleich einem Wasserstoffatom, einer geradkettigen oder verzweigten C1 bis C4-Alkylgruppe, einer C1- bis C4-Hydroxyalkylgruppe, einem gegebenenfalls substituierten aromatischen Carbozyklus oder einem gegebenenfalls substituierten aromatischen Heterocyclus; und R8" gleich einem zur Bildung einer natürlichen α-Aminosäure erforderlichen Restes (wie zum Beispiel dem (CH₂)₃-Guanidinylrest zur Bildung der α-Aminosäure Arginin, dem CH₂-Imidazoylrest zur Bildung der α-Aminosäure Histidin oder dem CH₂-Indolylrest zur Bildung der α-Aminosäure Tryptophan) ist.

Unter den Verbindungen der Formeln (III) bis (X) sind insbesondere die folgenden Verbindungen zu nennen: 1,3,3-Trimethyl-2-methylen-indolin sowie dessen Salze, 1,3,3,4-Tetramethyl-2-methylen-indolin sowie dessen Salze, 1,3,3,5-Tetramethyl-2-methylen-indolin sowie dessen Salze, 1,3,3,6-Tetramethyl-2-methylen-indolin sowie dessen Salze, 1,3,3,7-Tetramethyl-2-methylen-indolin sowie dessen Salze, 1,3,3,6,7-Pentamethyl-2-methylen-indolin sowie dessen Salze, 1,3,3,5,7-Pentamethyl-2-methylen-indolin sowie dessen Salze, 1,3,3,4,7-Pentamethyl-2-methylen-indolin sowie dessen Salze, 5-Chloro-1,3,3-trimethyl-2-methylen-indolin sowie dessen Salze, 5-Fluoro-1,3,3-trimethyl-2-methylen-indolin sowie dessen Salze, 5-Isopropyl-1,3,3-trimethyl-2-methylen-indolin sowie dessen Salze, 5-Hydroxy-1,3,3-trimethyl-2-methylen-indolin sowie dessen Salze, 5-Methoxy-1,3,3-trimethyl-2-methylen-indolin sowie dessen Salze, 5-Amino-1,3,3-trimethyl-2-methylen-indolin sowie dessen Salze, 5-N-acetylamino-1,3,3-trimethyl-2-methylen-indolin sowie dessen Salze, 5-Methoxy-6-N-acetylamino-1,3,3-trimethyl-2-methylen-indolin sowie dessen Salze, 5-Hydroxy-7-N-acetylamino-1,3,3-trimethyl-2-methylen-indolin sowie dessen Salze, 6-Hydroxy-1,3,3-trimethyl-2-methylen-indolin sowie dessen Salze, 6-Methoxy-1,3,3-trimethyl-2-methylen-indolin sowie dessen Salze 5-Methoxy-6-amino-1,3,3-trimethyl-2-methylen-indolin sowie dessen Salze, 5,6-Dihydroxy-1,3,3-trimethyl-2-methylen-indolin sowie dessen Salze, 5,6-Dimethoxy-1,3,3-trimethyl-2-methylen-indolin sowie dessen Salze, 4,5-Dihydroxy-1,3,3-trimethyl-2-methylen-indolin sowie dessen Salze, 5,7-Dihydroxy-1,3,3-trimethyl-2-methylen-indolin sowie dessen Salze, 5-Amino-6-methoxy-1,3,3-trimethyl-2-methylen-indolin sowie dessen Salze, 5-Amino-7-hydroxy-1,3,3-trimethyl-2-methylen-indolin sowie dessen Salze, 5-Hydroxy-7-amino-1,3,3-trimethyl-2-methylen-indolin sowie dessen Salze, 1-(2'-Hydroxyethyl)-3,3-dimethyl-2-methylen-indolin sowie dessen Salze, 1,3,3-Trimethyl-2-methylen-3H-benz[e]indol sowie dessen Salze und N-Ethyl-2-methylen-benzthiazol sowie dessen Salze;
wobei das 1,3,3-Trimethyl-2-methylen-indolin, 1,2,3,3-Tetramethyl-3H-indolium-chlorid, 1,2,3,3-Tetramethyl-3H-indolium-bromid, 1,2,3,3-Tetramethyl-3H-indolium-jodid, 1,2,3,3-Tetramethyl-3H-indolium-sulfat, 1,2,3,3-Tetramethyl-3H-indolium-hydrogensulfat, 1,2,3,3-Tetramethyl-3H-indolium-methylsulfat, 1,2,3,3-Tetramethyl-3H-indolium-hexafluorophosphat, 1,2,3,3-Tetramethyl-3H-indolium-hexafluoroantimonat, 1,2,3,3-Tetramethyl-3H-indolium-tetrafluoroborat, 1,2,3,3,5-Pentamethyl-3H-indolium-jodid, 1,2,3,3,7-Pentamethyl-3H-indolium-tetrafluoroborat, 1,2,3,3,6,7-Hexamethyl-3H-indolium-tetrafluoroborat, 1,2,3,3,5,7-Hexamethyl-3H-indolium-tetrafluoroborat, 1,2,3,3,4,7-Hexamethyl-3H-indolium-tetrafluoroborat, 5-Chloro-1,2,3,3-tetramethyl-3H-indolium-jodid, 5-Fluoro-1,2,3,3-tetramethyl-3H-indolium-jodid, 5-Isopropyl-1,2,3,3-tetramethyl-3H-indolium-jodid, 5-Methoxy-1,2,3,3-tetramethyl-3H-indolium-jodid, 5-Hydroxy-1,2,3,3-tetramethyl-3H-indolium-jodid, 1,2,3,3-Tetramethyl-3H-benz[e]indolium-chlorid, 1,2,3,3-Tetramethyl-3H-benz[e]indolium-bromid, 1,2,3,3-Tetramethyl-3H-benz[e]indolium-jodid, 1,2,3,3-Tetramethyl-3H-benz[e]indolium-sulfat, 1,2,3,3-Tetramethyl-3H-benz[e]indolium-hexafluorphosphat, 1,2,3,3-Tetramethyl-3H-benz[e]indolium-methylsulfat, 1,2,3,3-Tetramethyl-3H-benz[e]indolium-hexafluorantimonat, 1,2,3,3-Tetramethyl-3H-benz[e]indolium-tetrafluorborat, 1,2-Dimethylbenzthiazolium-jodid und N-Ethyl-2-methyl-benzthiazolium-jodid besonders bevorzugt sind.

Die in dem erfindungsgemässen Mittel verwendeten Schiffschen Basen der Formel (II) lassen sich nach literaturbekannten Standardsyntheseverfahren, wie sie beispielsweise in Houben-Weyl, "Methoden der organischen Chemie" Band 7, Teil 1, Seiten 453-460 (1954) oder Jerry March, "Advanced Organic Chemistry", 4th Edition (1992), Seiten 896-898 beschrieben werden, durch Umsetzung der entsprechenden Amine und Carbonylverbindungen herstellen.

Unter den Verbindungen der Formel (II) sind die folgenden Verbindungen besonders bevorzugt:
4-(((2-Hydroxyethyl)imino)methyl)-2-methoxyphenol,
5-(((2-Hydroxyethyl)imino)methyl)-2-methoxyphenol,
2,6-Dimethoxy-4-(((2-hydroxyethyl)imino)methyl)phenol,
4-(((2-Hydroxyethyl)imino)methyl)phenol,
1,2-Dihydroxy-4-(((2-hydroxyethyl)imino)methyl)benzol,
N,N-dimethyl-4-(((2-hydroxyethyl)imino)methyl)-anilin,
1,2-Dihydroxy-3-(((2-hydroxyethyl)imino)methyl)benzol,
4-(((3-Hydroxypropyl)imino)methyl)phenol,
2,6-Dimethoxy-4-(((3-Hydroxypropyl)imino)methyl)phenol,
4-(((2,3-Dihydroxypropyl)imino)methyl)phenol,
2,6-Dimethoxy-4-(((2,3-dihydroxypropyl)imino)methyl)phenol,
2-[(4-Hydroxy-benzyliden)-amino]-propan-1,3-diol,
2-[(4-Hydroxy-3,5-dimethoxy-benzyliden)-amino]-propan-1,3-diol,
4-(((2-Hydroxy-2-phenyl-ethyl)imino)methyl)phenol,
2,6-Dimethoxy-4-(((1-phenyl-2-hydroxy-ethyl)imino)methyl)phenol,
4-(((2-hydroxyphenyl)imino)methyl)phenol,
2,6-Dimethoxy-4-(((2-hydroxyphenyl)imino)methyl)phenol,
5-Guanidino-2-[(4-hydroxy-benzyliden)-amino]-pentansäure,
2-[(4-Dimethylamino-naphthalen-1-ylmethylen)-amino]-ethanol,
5-Guanidino-2-[(4-hydroxy-3,5-dimethoxy-benzyliden)-amino]-pentansäure, 2-[(4-Hydroxy-3,5-dimethoxy-benzyliden)-amino]-3-(3*H-*imidazol-4-yl)-propansäure, 2-[(4-Hydroxy-benzyliden)-amino]-3-(3*H-*imidazol-4-yl)-propansäure, 2-[(4-Hydroxy-3,5-dimethoxy-benzyliden)-amino]-3-(1*H-*indol-3-yl)-propansäure, 2-[(4-Hydroxy-benzyliden)-amino]-3-(1*H*-indol-3-yl)-propansäure, 2-(((2-Hydroxyethyl)imino)methyl)phenol,
1,2-Dihydroxy-3-(((2-hydroxyethyl)imino)methyl)benzol, 1,2,3-Trihydroxy-4-(((2-hydroxy-ethyl)imino)methyl)benzol, 1,2,3,4-Tetrahydroxy-5-(((2-hydroxyethyl)-imino)methyl)benzol und 1,2,4-Trihydroxy-4-(((2-hydroxyethyl)imino)-methyl)benzol.

Die Verbindungen der Formel (III) bis (X) und die Verbindungen der Formel (II) werden getrennt voneinander aufbewahrt und erst kurz vor der Anwendung miteinander vermischt.

Die Enamine der Formel (III) bis (X) sowie die Schiffsche Base der Formel (II) sind in der gebrauchsfertigen Komponente (A) jeweils in einer Gesamtmenge von etwa 0,01 bis 10 Gewichtsprozent, vorzugsweise etwa 0,1 bis 5 Gewichtsprozent, enthalten.

Die Zubereitungsform für die Komponenten (A1) und (A2) sowie des gebrauchsfertigen Färbemittels (A) kann beispielsweise eine Lösung, insbesondere eine wässrige oder wässrig-alkoholische Lösung, eine Creme, ein Gel oder eine Emulsion sein. Ihre Zusammensetzung stellt eine Mischung der Enamine der Formel (III) bis (X) beziehungsweise der Schiffschen Base der Formel (II) mit den für solche Zubereitungen üblichen Zusätzen dar.

Übliche in Färbemitteln verwendete Zusätze in Lösungen, Cremes, Emulsionen, Gelen oder Aerosolschäumen sind zum Beispiel Lösungsmittel wie Wasser, niedere aliphatische Alkohole, beispielsweise Ethanol, n-Propanol und Isopropanol oder Glykole wie Glycerin und 1,2-Propandiol, weiterhin Netzmittel oder Emulgatoren aus den Klassen der anionischen, kationischen, amphoteren oder nichtionogenen oberflächenaktiven Substanzen wie Fettalkoholsulfate, oxethylierte Fettalkoholsulfate, Alkylsulfonate, Alkylbenzolsulfonate, Alkyltrimethylmmoniumsalze, Alkylbetaine, oxethylierte Fettalkohle, oxethylierte Nonylphenole, Fettsäurealkanolamide, oxethylierte Fettalkohole, oxethylierte Nonylphenole, Fettsäurealkanolamide, oxethylierte Fettsäureester, ferner Verdicker wie höhere Fettalkohole, Stärke oder Cellulosederivate, Parfüme, Haarvorbehandlungsmittel, Konditionierer, Haarquellmittel, Konservierungsstoffe, weiterhin Vaseline, Paraffinöl und Fettsäuren sowie außerdem Pflegestoffe wie kationische Harze, Lanolinderivate, Cholesterin, Pantothensäure und Betain. Die erwähnten Bestandteile werden in den für solche Zwecke üblichen Mengen verwendet, zum Beispiel die Netzmittel und Emulgatoren in Konzentrationen von etwa 0,5 bis 30 Gewichtsprozent, die Verdicker in einer Menge von etwa 0,1 bis 25 Gewichtsprozent und die Pflegestoffe in einer Konzentration von etwa 0,1 bis 5,0 Gewichtsprozent.

Weiterhin kann das erfindungsgemäße Färbemittel gegebenenfalls zusätzlich übliche, physiologisch unbedenkliche, direktziehende Farbstoffe aus der Gruppe der Nitrofarbstoffe, Azofarbstoffe, Chinonfarbstoffe und Triphenylmethanfarbstoffe enthalten.

Diese direktziehenden Farbstoffe können in der Komponente (A1) und/oder der Komponente (A2) jeweils in einer Gesamtmenge von etwa 0,02 bis 20 Gewichtsprozent, vorzugsweise 0,2 bis 10 Gewichtsprozent, eingesetzt werden, wobei die Gesamtmenge an direktziehenden Farbstoffen in dem durch Vermischen der Komponenten (A1) und (A2) erhaltenen gebrauchsfertigen Färbemittel etwa 0,01 bis 10 Gewichtsrozent, vorzugsweise 0,1 bis 5 Gewichtsprozent, beträgt.

Der pH-Wert des gebrauchsfertigen Färbemittels (A) beträgt in der Regel 3 bis 12, vorzugsweise etwa 6 bis 11, wobei sich der pH-Wert des gebrauchsfertigen Färbemittels (A) bei der Mischung der sauer eingestellten Komponente (A1) mit der alkalisch eingestellten Komponente (A2) auf einen Wert einstellt, der durch die Säuremenge in der Komponente (A1) und die Alkalimenge in der Komponente (A2) sowie durch das Mischungsverhältnis dieser beiden Komponenten beinflußt wird.

Die Komponente (A1) weist vorzugsweise einen pH-Wert von etwa 1 bis 4,5, insbesondere 1,5 bis 3, auf, während die Komponente (A2) vorzugsweise einen pH-Wert von etwa 7,5 bis 12, insbesondere 8 bis 11, aufweist.

Zur Einstellung des für die Färbung gewünschten pH-Wertes der Komponenten (A1) und (A2) sowie des gebrauchsfertigen Färbemittels (A) können -falls erforderlich- alkalisierende Mittel, wie zum Beispiel Alkanolamine, Alkylamine, Alkalihydroxide oder Ammoniumhydroxid und Alkalicarbonate oder Ammoniumcarbonate, oder Säuren, wie zum Beispiel Milchsäure, Essigsäure, Weinsäure, Phosphorsäure, Salzsäure, Zitronensäure, Ascorbinsäure oder Borsäure, verwendet werden.

Das gebrauchsfertige Färbemittel wird unmittelbar vor der Anwendung durch Vermischen der Komponenten (A1) und (A2) hergestellt und sodann auf die Faser, insbesondere menschliche Haare, aufgetragen. Je nach gewünschter Farbtiefe läßt man diese Mischung 5 bis 60 Minuten, vorzugsweise 15 bis 30 Minuten, bei einer Temperatur von etwa 20 bis 50 °C, insbesondere bei 30 bis 40 °C einwirken. Anschließend wird die Faser mit Wasser gespült und gegebenenfalls mit einem Shampoo gewaschen.

Ein weiterer Gegenstand der vorliegenden Erfindung ist ein Mehrkomponenten-Kit, bestehend aus einem Mittel der Komponente (A1), einem Mittel der Komponente (A2), sowie gegebenenfalls einem Mittel zur Einstellung des pH-Wertes. Selbstverständlich können auch die Mittel der Komponenten (A1) und (A2) aus mehreren Einzelkomponenten bestehen, welche erst unmittelbar vor der Anwendung miteinander vermischt werden. Besonders bevorzugt ist jedoch ein 2-Komponenten-Kit, bestehend aus einem Mittel der Komponente (A1) und einem Mittel der Komponente (A2).

Das erfindungsgemässe Färbemittel ist sehr lagerstabil und ermöglicht eine schonende, gleichmässige und dauerhafte Färbung der Fasern, insbesondere von Keratinfasern, wie zum Beispiel menschlichen Haaren, wobei die erhaltene Färbung nicht nachdunkelt. Überraschenderweise können diese Färbungen zu einem beliebigen Zeitpunkt durch Sulfite auf einfache und schonende Weise wieder entfernt werden.

Ein weiterer Gegenstand der vorliegenden Erfindung ist daher ein Verfahren zum temporären Färben von Fasern, bei dem die Faser zunächst mit dem erfindungsgemässen Färbemittel (A) gefärbt wird, und die Färbung zu einem beliebigen späteren Zeitpunkt mit einem als entfärbendes Agenz mindestens ein Sulfit enthaltenden Entfärbemittel wieder entfernt wird; sowie ein Mehrkomponenten-Kit zum Färben und späteren Entfärben von Fasern, enthaltend das erfindungsgemässe Färbemittel (A) sowie ein sulfithaltiges Entfärbemittel (B).

Als Sulfit können beispelsweise Ammoniumsulfit, Alkalisulfite oder Erdalkalisulfite verwendet werden, wobei Natriumsulfit und Ammoniumsulfit besonders bevorzugt sind. Die Gesamtmenge an Sulfiten in dem Entfärbemittel beträgt etwa 0,1 bis 10 Gewichtsprozent, vorzugsweise 2 bis 5 Gewichtsprozent.

Das Entfärbemittel kann als wässrige oder wässrig-alkoholische Lösung, als Gel, Creme, Emulsion oder Schaum vorliegen, wobei das Entfärbemittel sowohl in Form eines Einkomponentenpräparats als auch in Form eines Mehrkomponentenpräparates konfektioniert sein kann. Das Entfärbemittel kann neben der Pulverform zum Schutz vor Staubbildung auch als Tablette - auch Brausetablette - oder Granulat konfektioniert sein. Hieraus wird dann vor der Anwendung mit kaltem oder warmem Wasser, gegebenenfalls unter Zusatz eines oder mehrerer der nachfolgend genannten Hilfsmittel, das Entfärbemittel hergestellt. Es ist jedoch auch möglich, daß diese Hilfsmittel (sofern sie in fester Form vorliegen) bereits in dem Entfärbepulver oder Entfärbegranulat beziehungsweise der Brausetablette enthalten sind. Durch Benetzung des Pulvers durch Öle oder Wachse kann zusätzlich die Staubbildung vermindert werden.

Das Entfärbemittel kann zusätzliche Hilfsmittel, wie zum Beispiel Lösungsmittel wie Wasser, niedere aliphatische Alkohole, beispielsweise Ethanol, n-Propanol und Isopropanol, Glykolether oder Glykole wie Glycerin und insbesondere 1,2-Propandiol, weiterhin Netzmittel oder Emulgatoren aus den Klassen der anionischen, kationischen, amphoteren oder nichtionogenen oberflächenaktiven Substanzen wie Fettalkoholulfate, oxethylierte Fettalkoholsulfate, Alkylsulfonate, Alkylbenzolsulfonate, Alkyltrimethylammoniumsalze, Alkylbetaine, oxethylierte Fettalkohle, oxethylierte Nonylphenole, Fettsäurealkanol-amide, oxethylierte Fettalkohole, oxethylierte Nonylphenole, Fettsäurealkanolamide, oxethylierte Fettsäureester, ferner Verdicker wie höhere Fettalkohole, Stärke oder Cellulosederivate, Parfüme, Haarvorbehandlungsmittel, Konditionierer, Haarquellmittel, Konservierungsstoffe, Vaseline, Paraffinöl und Fettsäuren sowie außerdem Pflegestoffe wie kationische Harze, Lanolinderivate, Cholesterin, Pantothensäure und Betain, enthalten.

Der pH-Wert des Entfärbemittels beträgt etwa 3 bis 8, insbesondere 4 bis 7. Erforderlichenfalls kann der gewünschte pH-Wert durch Zugabe von geeigneten Säuren, beispielsweise α-Hydroxycarbonsäuren wie Milchsäure, Weinsäure, Zitronensäure oder Äpfelsäure, Phosphorsäure, Essigsäure, Glycolsäure Salicylsäure, Glutathion oder Gluconsäurelacton, oder aber alkalisierenden Mitteln wie Alkanolaminen, Alkylaminen, Alkalihydroxiden, Ammoniumhydroxid, Alkalicarbonaten, Ammoniumcarbonaten oder Alkaliphosphaten, eingestellt werden.

Zur Entfärbung der mit dem erfindungsgemässen Mittel gefärbten Faser wird das Entfärbemittel auf die Faser aufgetragen und bei etwa 20 bis 50 °C für einen Zeitraum von etwa 5 bis 60 Minuten, insbesondere 15 bis 30 Minuten, einwirken gelassen. Nach Beendigung der Einwirkungszeit des Entfärbemittels wird das Haar mit Wasser gespült, gegebenenfalls mit einem Shampoo gewaschen und abschliessend getrocknet.

Die nachfolgenden Beispiele sollen den Gegenstand näher erläutern, ohne ihn auf diese Beispiele zu beschränken.

### Beispiele

### Beispiele 1.1 bis 1.19:Synthese der Schiffschen Basen der Formel (II)

### Allgemeine Synthesevorschriften

### Vorschrift A:

Die Carbonylverbindung wird bei 80 °C in Toluol gelöst und mit 1 Äquivalent des primären Amins versetzt. Man erhitzt am Wasserabscheider unter Rückfluss. Wird kein Reaktionswasser mehr abgeschieden, destilliert man das Lösungsmittel unter vermindertem Druck ab. Der gebildete Rückstand wird durch Umkristallisation gereinigt.

### Vorschrift B:

Die Carbonylverbindung wird in Chloroform bei Raumtemperatur gelöst und mit 1 Äquivalent des primären Amins versetzt. Man rührt etwa 1 Stunde lang bei Raumtemperatur. Falls die Schiffsche Base ausfällt wird der gebildete Feststoff filtriert, andernfalls destilliert man das Lösungsmittel unter vermindertem Druck ab. Der filtrierte Feststoff bzw. der erhaltene Rückstand wird durch Umkristallisation gereinigt.

### Vorschrift C:

Die Carbonylverbindung wird in tert.Butyl-methylether bei 20 bis 55 °C gelöst und mit 1 Äquivalent des primären Amins versetzt. Man rührt 1 bis 3 Stunden lang bei Raumtemperatur oder erwärmt 1 bis 3 Stunden lang unter Rückfluss. Das Lösungsmittel wird unter vermindertem Druck entfernt und der gebildete Feststoff durch Umkristallisation gereinigt.

### Beispiel 1.1: Synthese von 4-(((2-Hydroxyethyl)imino)methyl)-2-methoxyphenol

Die Herstellung erfolgt gemäss der Synthesevorschrift A, wobei 6,25 g 4-Hydroxy-3-methoxybenzaldehyd und 2,50 ml Monoethanolamin in 250 ml Toluol umgesetzt werden. Der resultierende Rückstand wird aus Essigsäureethylester umkristallisiert.
Ausbeute: 6,41 g (80% der Theorie) 4-(((2-Hydroxyethyl)imino)methyl)-2-methoxyphenol
Schmelzpunkt: 129°C
¹H-NMR (D₆-DMSO): δ= 3,52-3,58 ppm (m, 2H); 3,58-3,64 ppm (m, 2H), 3,78 ppm (s, 3H); 6,79 ppm (d, ³J = 8 Hz, 1H); 7,08 ppm (dd, ³J = 8,0 Hz, ⁴J = 2,0 Hz, 1H); 7,32 ppm (d, ³J = 2,0 Hz, 1H); 8,14 ppm (s, 1H). ¹³C-NMR(D₆-DMSO): δ= 55,5 ppm (q); 61,0 ppm (t); 63,1 ppm (t); 109,9 ppm (d); 115,2 ppm (d); 122,8 ppm (d); 127,8 ppm (s); 147,9 ppm (s); 149,5 ppm (s); 161,3 ppm (d).
El-Massenspektrum:
195 (26, M⁺); 164 (71); 150 (7); 137 (100); 122 (16); 104 (41); 94 (18); 77 (18); 65 (26); 51 (21).
Elementaranalyse:

| C₁₀H₁₃NO₃(195,22) | | %C | %H | %N |
|---|---|---|---|---|
| | ber.: | 61,53 | 6,71 | 7,17 |
| | gef.: | 61,0 | 6,77 | 7,06 |

### Beispiel 1.2: Synthese von 5-(((2-Hydroxyethyl)imino)methyl)-2-methoxyphenol

Die Herstellung erfolgt gemäss der Synthesevorschrift A, wobei 5,0 g 3-Hydroxy-4-methoxybenaldehyd und 2,0 ml Monoethanolamin in 150 ml Toluol umgesetzt werden. Der resultierende Rückstand wird aus Chloroform umkristallisiert.
Ausbeute: 5,61 g (87% der Theorie) 5-(((2-Hydroxyethyl)imino)methyl)-2-methoxyphenol.
Schmelzpunkt: 114-116°C
¹H-NMR (MeOD): δ= 3,70 ppm (t, ³J = 5,5 Hz, 2H); 3,84 ppm (t, ³J = 5,5 Hz, 2H); 3,93 ppm (s, 3H); 7.00 ppm (d, ³J = 8,5 Hz, 1H); 7,21 ppm (dd, ³J = 8,5 Hz, ⁴J = 2,0 Hz, 1H); 7,31 ppm (d, ⁴J = 2,0 Hz, 1H); 8,22 ppm (s, 1H).
¹³C-NMR (MeOD): δ= 56,4 ppm (q); 62,4 ppm (t); 64,0 ppm (t); 112,1 ppm (d); 114,9 ppm (d); 122,9 ppm (d); 130,3 ppm (s); 147,9 ppm (s); 151,9 ppm (s); 165,5 ppm (d).
FAB-Massenspektrum: M⁺= 169,2 (100% rel. Intensität)
Elementaranalyse:

| C₁₀H₁₃NO₃ (195,22) | | %C | %H | %N |
|---|---|---|---|---|
| | ber.: | 61,53 | 6,71 | 7,18 |
| | gef.: | 61,76 | 6,94 | 7,06 |

### Beispiel 1.3: Synthese von 2,6-Dimethoxy-4-(((2-hydroxyethyl)imino)-methyl)phenol

Die Herstellung erfolgt gemäss der Synthesevorschrift B, wobei 5,0 g 3,5-Dimethoxy-4-hydroxybenzaldehyd und 1,70 ml Monoethanolamin in 50 ml CHCl₃ umgesetzt werden. Der resultierende Rückstand wird aus Acetonitril umkristallisiert.
Ausbeute: 6,19 g (84% der Theorie 2,6-Dimethoxy-4-(((2-hydroxyethyl)-imino)methyl)phenol
¹H-NMR (MeOD): δ= 3,71 ppm (t, ³J = 5,5 Hz, 2H); 3,85 ppm (t, ³J = 5,5 Hz, 2H), 3,89 ppm (s, 6H); 7,11 ppm (s, 2H); 8,16 ppm (s, 1H).
¹³C-NMR (D₆-DMSO): δ= 56,1 ppm (q); 61,1 ppm (t); 63,1 ppm (t); 105,6 ppm (d); 126,5 ppm (s); 138,9 ppm (s); 148,2 ppm (s); 161,6 ppm (d).
El-Massenspektrum: 225 (14, M⁺); 194 (48); 180 (9); 179 (10); 167 (14); 162 (48); 148 (28); 134 (100); 107 (50); 79 (32); 65 (18).
Elementaranalyse:

| C₁₁H₁₅NO₄ x 1 CH₃CN (266,30) | | %C | %H | %N |
|---|---|---|---|---|
| | ber.: | 58,63 | 6,81 | 10,52 |
| | gef.: | 57,85 | 6,91 | 9,49 |

### Beispiel 1.4: Synthese von 4-(((2-Hydroxyethyl)imino)methyl)phenol

Die Herstellung erfolgt gemäss der Synthesevorschrift C, wobei 2,0 g 4-hydroxybenzaldehyd und 1,0 ml Monoethanolamin in 30 ml tert.Butylmethylether umgesetzt werden. Der resultierende Rückstand wird aus 1-Butanol umkristallisiert.
Ausbeute: 2,20 g (83% der Theorie) 4-(((2-Hydroxyethyl)imino)methyl)-phenol
¹H-NMR (MeOD): δ= 3,69 ppm (t, ³J = 5,5 Hz, 2H); 3,83 ppm (t, ³J = 5,5 Hz, 2H), 6,84 ppm (∼d, ³J ∼ 8,5 Hz, 2H); 7,64 ppm (∼d, ³J ∼ 8,5 Hz, 2H); 8,23 ppm (s, 1H).
¹³C-NMR (MeOD): δ= 62,3 ppm (t); 63,3 ppm (t); 117,0 ppm (d); 127,4 ppm (s); 131,7 ppm (d); 163,4 ppm (s); 165,5 ppm (d).
El-Massenspektrum: 165 (31, M⁺); 134 (99); 120 (22); 107 (100); 93 (8); 77 (62); 65 (22); 51 (12).
Elementaranalyse:

| C₉H₁₁NO₂ (165,19) | | %C | %H | %N |
|---|---|---|---|---|
| | ber.: | 65,44 | 6,71 | 8,48 |
| | gef.: | 65,74 | 6,94 | 8,36 |

### Beispiel 1.5: Synthese von 1,2-Dihydroxy-4-(((2-hydroxyethyl)imino)-methyl)benzol

Die Herstellung erfolgt gemäss der Synthesevorschrift C, wobei 5,0 g 3,4-Dihydroxybenzaldehyd und 2,20 ml Monoethanolamin in 100 ml tert.Butyl-methylether umgesetzt werden. Der resultierende Rückstand wird in heissem Isopropanol gelöst; man lässt erkalten und verwirft den gebildeten Feststoff. Das Filtrat wird anschliessend unter vermindertem Druck eingedampft und der gebildete Rückstand aus 1-Butanol umkristallisiert.
Ausbeute: 2,70 g (41 % der Theorie) 1,2-Dihydroxy-4-(((2-hydroxyethyl)-imino)methyl)benzol
Schmelzpunkt: 103-107 °C
¹H-NMR (MeOD): δ= 3,67 ppm (t, ³J = 5,5 Hz, 2H); 3,82 ppm (t, ³J = 5,5 Hz, 2H); 6,77 ppm (d, ³J = 8 Hz, 1H); 7,14 ppm (d, ³J = 8 Hz, 1H); 7,26 ppm (s, 1H); 8,09 ppm (s, 1H).
¹³C-NMR (MeOD): δ= 61,4 ppm (t); 62,2 ppm (t); 113,8 ppm (d); 116,8 ppm (d); 124,8 ppm (s); 126,5 ppm (d); 147,9 ppm (s); 156,1 ppm (s); 165,5(d).

### Beispiel 1.6: Synthese von N,N-dimethyl-4-(((2'-hydroxyethyl)imino)-methyl)anilin

Die Herstellung erfolgt gemäss der Synthesevorschrift C, wobei 5,0 g 4-(Dimethylamino)benzaldehyd und 2,0 ml Monoethanolamin in 50 ml tert.Butyl-methylether umgesetzt werden. Der resultierende Rückstand wird aus Acetonitril umkristallisiert.
Ausbeute: 5,21 g (81 % der Theorie) N,N-dimethyl-4-(((2'-hydroxyethyl)-imino)methyl-anilin
Schmelzpunkt: 103-105 °C
¹H-NMR (MeOD): δ= 3,03 ppm (s, 6H); 3,67 ppm (t, ³J = 5,5 Hz, 2H); 3,82 ppm (t, ³J = 5,5 Hz, 2H); 6,77 ppm (∼d, ∼³J = 9 Hz, 2H); 7,62 ppm (∼d, ∼³J = 9 Hz, 1H); 8,19 ppm (s, 1H).
El-Massenspektrum: 192 (85, M⁺); 161 (100); 146 (70); 134 (88); 117 (56); 91 (30); 81 (25);
Elementaranalyse:

| C₁₁H₁₆N₂O (192,26) | | %C | %H | %N |
|---|---|---|---|---|
| | ber.: | 68,72 | 8,39 | 14,57 |
| | gef.: | 68,90 | 8,60 | 14,58 |

### Beispiel 1.7: Synthese von 1,2-Dihydroxy-3-(((2-hydroxyethyl)imino)-methyl)benzol

Die Herstellung erfolgt gemäss der Synthesevorschrift C, wobei 2,0 g 2,3-Dihydroxybenzaldehyd und 0,9 ml Monoethanolamin in 30 ml tert.Butyl-methylether umgesetzt werden. Der resultierende Rückstand wird aus Essigsäureethylester umkristallisiert.
Ausbeute: 2,08 g (79 % der Theorie) 1,2-Dihydroxy-3-(((2-hydroxyethyl)-imino)methyl)benzol
Schmelzpunkt: 112-116°C
¹H-NMR (MeOD): δ= 3,75 ppm (t, ³J = 5,0 Hz, 2H); 3,84 ppm (t, ³J = 5,0 Hz, 2H); 6,60-6,66 ppm (m, 1H); 6,84-6,90 ppm (m, 2H); 8,40 ppm (s, 1H).
Elementaranalyse:

| C₉H₁₁NO₃ (181,19) | | %C | %H | %N |
|---|---|---|---|---|
| | ber.: | 59,66 | 6,12 | 7,73 |
| | gef.: | 59,63 | 6,19 | 7,60 |

### Beispiel 1.8: Synthese von 4-(((3-Hydroxypropyl)imino)methyl)phenol

Die Herstellung erfolgt gemäss der Synthesevorschrift C, wobei 5,0 g 4-Hydroxybenzaldehyd und 3,08 g 3-Amino-1-propanol in 70 ml tert.Butylmethylether umgesetzt werden. Der resultierende Rückstand wird aus Acetonitril umkristallisiert.
Ausbeute: 5,62 g (77 % der Theorie) 4-(((3-Hydroxypropyl)imino)methyl)-phenol
Schmelzpunkt: 110-115 °C
¹H-NMR (MeOD): δ= 1,88-1,97 ppm (m, 2H); 3,65-3,70 ppm (m, 4H); 6,84 ppm (∼d, ∼³J = 9 Hz, 2H); 7,62 ppm (-d, ∼³J = 9 Hz, 2H); 8,23 ppm (s, 1H).
Elementaranalyse:

| C₁₀H₁₃NO₂ (179,22) | | %C | %H | %N |
|---|---|---|---|---|
| | ber.: | 67,02 | 7,31 | 7,82 |
| | gef.: | 67,30 | 7,59 | 7,82 |

### Beispiel 1.9: Synthese von 2,6-Dimethoxy-4-(((3-hydroxypropyl)-imino)methyl)phenol

Die Herstellung erfolgt gemäss der Synthesevorschrift C, wobei 5,0 g 3,5-Dimethoxy-4-hydroxybenzaldehyd und 2,06 g 3-Amino-1-propanol in 100 ml tert.Butyl-methylether umgesetzt werden. Der resultierende Rückstand wird aus Acetonitril umkristallisiert.
Ausbeute: 2,20 g (33% der Theorie) 2,6-Dimethoxy-4-(((3-hydroxypropyl)-imino)methyl)phenol
Schmelzpunkt:143-146 °C.
¹H-NMR (MeOD): δ= 1,90-2,00 ppm (m, 2H); 3,66-3,72 ppm (m, 4H); 3,89 ppm (s, 6H); 7,09 ppm (s, 2H); 8,16 ppm (s, 1H).
El-Massenspektrum: 239 (26, M⁺); 209 (30); 194 (100); 180 (52); 164 (32);134 (44); 120 (20); 107 (28); 94 (20); 79 (26):
Elementaranalyse:

| C₁₂H₁₇NO₄(239,27) | | %C | %H | %N |
|---|---|---|---|---|
| | ber.: | 60,24 | 7,16 | 5,85 |
| | gef.: | 59,99 | 7,44 | 5,77 |

### Beispiel 1.10: Synthese von 4-(((2,3-Dihydroxypropyl)imino)-methyl)phenol

Die Herstellung erfolgt gemäss der Synthesevorschrift C, wobei 5,0 g 4-Hydroxybenzaldehyd und 3,73 g 3-Amino-1,2-propandiol in 70 ml tert.Butyl-methylether umgesetzt werden. Der resultierende Rückstand wird aus Toluol umkristallisiert.
Ausbeute: 6,75 g (84% der Theorie) 4-(((2,3-Dihydroxypropyl)imino)-methyl)phenol
Schmelzpunkt:125-131 °C.
¹H-NMR (MeOD): δ= 3,45-3,55 ppm (m, 1H); 3,55-3,70 ppm (m, 2H); 3,75-3,85 ppm (m, 1H); 3,85-4,0 ppm (m, 1H); 6,84 ppm (∼d, ∼³J = 9 Hz, 2H); 7,64 ppm (∼d, ∼³J = 9 Hz, 2H) 8,23 ppm (s, 1H).
Elementaranalyse:

| C₁₀H₁₃NO₃(195,22) | | %C | %H | %N |
|---|---|---|---|---|
| | ber.: | 61,53 | 6,71 | 7,18 |
| | gef.: | 61,62 | 6,89 | 7,10 |

### Beispiel 1.11: Synthese von 2,6-Dimethoxy-4-(((2,3-dihydroxypropyl)-imino)methyl)phenol

Die Herstellung erfolgt gemäss der Synthesevorschrift B, wobei 5,0 g 3,5-Dimethoxy-4-hydroxybenzaldehyd und 2,50 g 3-Amino-1,2-propandiol in 50 ml CHCl₃ umgesetzt werden. Der resultierende Rückstand wird aus Isopropanol umkristallisiert.
Ausbeute: 3,12 g (45% der Theorie) 2,6-Dimethoxy-4-(((2,3-dihydroxypropyl)imino)methyl)phenol
Schmelzpunkt: 124-128°C
¹H-NMR (MeOD): δ= 3,48-3,60 ppm (m, 1H); 3,60-3,70 ppm (m, 2H); 3,75-4,0 ppm (m, 2H); 3,90 ppm (s, 6H); 7,12 ppm (s, 2H); 8,17 ppm (s, 1H).
Elementaranalyse:

| C₁₂H₁₇NO₅ (255,27) | | %C | %H | %N |
|---|---|---|---|---|
| | ber.: | 55,46 | 6,71 | 5,49 |
| | gef.: | 56,49 | 6,83 | 5,35 |

### Beispiel 1.12: Synthese von 2-[(4-Hydroxy-benzyliden)-amino]-propan-1,3-diol

Die Herstellung erfolgt gemäss der Synthesevorschrift A, wobei 2,44 g 4-Hydroxybenzaldehyd und 1,82 g 2-Amino-1,3-propandiol in 80 ml Toluol umgesetzt werden. Der resultierende Rückstand wird aus Methanol umkristallisiert.
Ausbeute: 0,48 g (12% der Theorie) 2-[(4-Hydroxy-benzyliden)-amino]-propan-1,3-diol
Schmelzpunkt: 196-199 °C
¹H-NMR (MeOD/D₆-DMSO): δ = 3,40-3,60 ppm (m, 1H); 3,65-3,80 ppm (m, 2H); 3,80-3,95 ppm (m, 2H); 6,93 ppm (m, 2H); 7,75 ppm (m, 2H); 8,35 ppm (s, 1H).
Elementaranalyse:

| C₁₀H₁₃NO₃ (195,22) | | %C | %H | %N |
|---|---|---|---|---|
| | ber.: | 61,53 | 6,71 | 7,18 |
| | gef.: | 61,48 | 6,92 | 6,90 |

### Beispiel 1.13: Synthese von 2-[(4-Hydroxy-3,5-dimethoxybenzyliden)-amino]-propan-1,3-diol

Die Herstellung erfolgt gemäss der Synthesevorschrift A, wobei 2,0 g 3,5-Dimethoxy-4-hydroxybenzaldehyd und 1,0 g 2-Amino-1,3-propandiol in 80 ml Toluol umgesetzt werden. Der resultierende Rückstand wird aus Acetonitril umkristallisiert.
Ausbeute: 2,10 g (75% der Theorie) 2-[(4-Hydroxy-3,5-dimethoxybenzyliden)-amino]-propane-1,3-diol
¹H-NMR (MeOD): δ= 3,40-3,60 ppm (m, 1H); 3,60-3,75 ppm (m, 2H); 3,75-3,90 ppm (m, 2H); 3,92 ppm (s, 6H), 7,17 ppm (s, 2H); 8,24 ppm (s, 1H).
ES-Massenspektrum: (M⁺-1) = 254.1 (100% rel. Intensität)

### Beispiel 1.14: Synthese von 4-(((2-Hydroxy-2-phenyl-ethyl)imino)-methyl)phenol

Die Herstellung erfolgt gemäss der Synthesevorschrift A, wobei 4,27 g 4-Hydroxybenzaldehyd und 4,80 g 2-Amino-1-phenylethanol in 100 ml Toluol umgesetzt werden. Der resultierende Rückstand wird aus Toluol umkristallisiert.
Ausbeute: 7,95 g (94% der Theorie) 4-(((2-Hydroxy-2-phenyl-ethyl)imino)-methyl)phenol
Schmelzpunkt: 165-169 °C
¹H-NMR (MeOD): δ= 3,65-3,80 ppm (m, 1H); 3,80-3,95 ppm (m, 1H); 4,95-5,05 ppm (m, 1H); 6,83 ppm (-d, ∼³J = 9 Hz, 2H); 7,22-7,50 ppm (m, 5H); 7,62 ppm (∼d, ∼³J = 9 Hz, 2H); 8,13 ppm (s, 1H)
ES-Massenspektrum: (M⁺-1) = 240.1 (100% rel. Intensität)
Elementaranalyse:

| C₁₅H₁₅NO₂(241,29) | | %C | %H | %N |
|---|---|---|---|---|
| | ber.: | 74,67 | 6,27 | 5,81 |
| | gef.: | 74,73 | 6,42 | 5,56 |

### Beispiel 1.15: Synthese von 2,6-Dimethoxy-4-(((1-phenyl-2-hydroxyethyl)imino)methyl)phenol

Die Herstellung erfolgt gemäss der Synthesevorschrift A, wobei 2,66 g 3,5-Dimethoxy-4-hydroxybenzaldehyd und 2,0 g (R)-2-Amino-2-phenylethanol in 80 ml Toluol umgesetzt werden. Der resultierende Rückstand wird aus Toluol/Petrolether (1:3) umkristallisiert.
Ausbeute: 4,05 g (92% der Theorie) 2,6-Dimethoxy-4-(((1-phenyl-2-hydroxy-ethyl)imino)methyl)phenol.
Schmelzpunkt: 58-62°C
¹H-NMR (MeOD): δ= 3,85-4,0 ppm (m, 2H); 3,90 ppm (s, 6H); 4,48 ppm (m, 1H); 7,10-7,50 ppm (m, 7H); 8.32 ppm (s, 1H)
ES-Massenspektrum: (M⁺-1) = 300,10 (100% rel. Intensität)
Elementaranalyse:

| C₁₇H₁₉NO₄ (301,35) | | %C | %H | %N |
|---|---|---|---|---|
| | ber.: | 67,76 | 6,36 | 4,65 |
| | gef.: | 67,48 | 6,57 | 4,15 |

### Beispiel 1.16:

### Synthese von 4-(((2-Hydroxyphenyl)imino)methyl)phenol

Die Herstellung erfolgt gemäss der Synthesevorschrift A, wobei 5,0 g 4-Hydroxybenzaldehyd und 4,47 g 2-Aminophenol in 100 ml Toluol umgesetzt werden. Der resultierende Rückstand wird aus Toluol umkristallisiert.
Ausbeute: 8,51 g (97% der Theorie) 4-(((2-Hydroxyphenyl)imino)-methyl)phenol.
Schmelzpunkt: 145-149 °C
¹H-NMR (MeOD): δ= 6,80-7,00 ppm (m, 4H); 7,05-7,25 ppm (m, 2H); 7,80-7,90 ppm (m, 2H); 8,56 ppm (s, 1H)
El-Massenspektrum: 213 (76, M⁺); 212 (92); 120 (100); 107 (12); 93 (30); 77 (22); 65 (64); 51 (24);
Elementaranalyse:

| C₁₃H₁₁NO₂ (213,24) | | %C | %H | %N |
|---|---|---|---|---|
| | ber.: | 73,23 | 5,20 | 6,57 |
| | gef.: | 72,98 | 5,29 | 6,25 |

### Beispiel 1.17: Synthese von 2,6-Dimethoxy-4-(((2-hydroxyphenyl)-imino)methyl)phenol

Die Herstellung erfolgt gemäss der Synthesevorschrift A, wobei 4,92 g 3,5-Dimethoxy-4-hydroxybenzaldehyd und 2,95 g 2-Aminophenol in 100 ml Toluol umgesetzt werden. Der resultierende Rückstand wird aus Toluol umkristallisiert.
Ausbeute: 6,24 g (83% der Theorie) 2,6-Dimethoxy-4-(((2-hydroxyphenyl)-imino)methyl)phenol
¹H-NMR (MeOD): δ = 3,94 ppm (s, 6H); 6,83-6,98 ppm (m, 2H); 7,05-7,22 ppm (m, 2H); 7,33 ppm (s, 2H); 8,52 ppm (s, 1H)
El-Massenspektrum: 273 (75, M⁺); 256 (10); 170 (10); 154 (52); 139 (26); 120 (100); 93 (30); 77 (18); 65 (44)
Elementaranalyse:

| C₁₅H₁₅NO₄ (273,29) | | %C | %H | %N |
|---|---|---|---|---|
| | ber.: | 65,93 | 5,53 | 5,13 |
| | gef.: | 66,11 | 5,62 | 5,00 |

### Beispiel 1.18: Synthese von 5-Guanidino-2-[(4-hydroxy-benzyliden)-amino]-pentansäure

Die Herstellung erfolgt gemäss der Synthesevorschrift A, wobei 3,0 g 4-Hydroxybenzaldehyd und 4,28 g L-Arginin in 100 ml Toluol umgesetzt werden. Der resultierende Rückstand wird aus Methanol umkristallisiert.
Ausbeute: 2,29 g (25% der Theorie) 5-Guanidino-2-[(4-hydroxybenzyliden)-amino]-pentansäure.
¹H-NMR (MeOD): δ = 1,58-1,75 ppm (m, 2H); 1,83-2,10 ppm (m, 2H); 3,15-3,28 ppm (m, 2H); 3,87 ppm (m, 1H); 6,81 ppm (∼d, ∼³J = 9 Hz, 2H); 7,67 ppm (∼d, ∼³J = 9 Hz, 2H); 8,19 ppm (s, 1H).
FAB-Massenspektrum: 279,0 (M⁺, 30% rel. Intensität);

### Beispiel 1.19: Synthese von 2-[(4-Dimethylamino-naphthalen-1-ylmethylen)-amino]-ethanol

Die Herstellung erfolgt gemäss der Synthesevorschrift B, wobei 3,0 g 4-(Dimethylamino)-Naphthalin-1-carboxaldehyd und 0,92 g Monoethanolamin in 20 ml Chloroform umgesetzt werden. Nach ca. 7 h Rühren bei Raumtemperatur wird die Reaktionslösung vollständig eingeengt. Der resultierende Rückstand, ein Öl, ist gemäss Analysendaten reines Produkt.
Ausbeute: 3,60 g (99% der Theorie) 2-[(4-Dimethylamino-naphthalen-1-ylmethylen)-amino]-ethanol.
¹H-NMR (DMSO): δ = 2,86 ppm (s, 6H); 3,71 ppm (m, 4H); 4,62 ppm (s, br, 1H); 7.11 ppm (d, ³J = 8 Hz, 1H); 7,50-7,60 ppm (m, 2H); 7,78 ppm (d, ³J = 8 Hz, 1H); 8,16 ppm (∼d, ∼³J = 9 Hz, 1H); 8,78 ppm (s, 1H); 9,19 ppm (∼d, ∼³J = 9 Hz, 1H);
El-Massenspektrum: 242 (100, M⁺); 227 (10); 211 (50); 196 (40); 167 (22); 154 (10); 139 (12); 127 (8); 115 (8);
Elementaranalyse:

| C₁₅H₁₈N₂O x 0,7 H₂O (254.93): | | %C | %H | %N |
|---|---|---|---|---|
| | ber.: | 70,67 | 7,67 | 10,99 |
| | gef.: | 70,58 | 7,54 | 11,04 |

### Beispiele 2.1 bis 2.20: Haarfärbemittel

### Komponente (A1)

Enamin der Formel (III) bis (X) Mengenangaben gemäß Tabelle 1

| | |
|---|---|
| 6-O-Palmitoyl-L-ascorbinsäure | 0,3 g |
| Cetylstearylalkohol | 12,0 g |
| Laurylethersulfat, 28%ige wässrige Lösung | 10,0 g |
| Ethanol | 23,0 g |
| Wasser, vollentsalzt | ad 100,0 g |

Der Cetylstearylalkohol wird bei 80°C geschmolzen. Das Laurylethersulfat und 95 % des Wassers werden auf 80°C erhitzt und zum geschmolzenen Cetylstearylalkohol gegeben und gerührt bis sich eine Creme ergibt. Bei Raumtemperatur wird die Verbindung der Formel (I) zusammen mit dem Ethanol, dem restlichen Wasser und der 6-O-Palmitoyl-L-ascorbinsäure zugegeben. Der pH des Mittels ist in Tabelle 1 angegeben.

### Komponente (A2)

Schiffsche Base der Formel (II) Mengenangaben gemäß Tabelle 1

| | |
|---|---|
| Cetylstearylalkohol | 12,0 g |
| Laurylethersulfat, 28%ige wässrige Lösung | 10,0 g |
| Ethanol | 23,0 g |
| Wasser, vollentsalzt | ad 100,0 g |

Der Cetylstearylalkohol wird bei 80 °C geschmolzen. Das Laurylethersulfat und 95 % des Wassers werden auf 80 °C erhitzt und zum geschmolzenen Cetylstearylalkohol gegeben und gerührt bis sich eine Creme ergibt. Bei Raumtemperatur wird die Verbindung der Formel (III) bis (X) zusammen mit dem Ethanol und dem restlichen Wasser zugegeben. Der pH-Wert der Creme wird mit einem geeigneten primären Amin oder mit 10 %iger Natronlauge auf den jeweils in Tabelle 1 angegebenen pH-Wert eingestellt.

Die Komponente (A1) und die Komponente (A2) werden im Verhältnis 1:1 miteinander vermischt. Das so erhaltene gebrauchsfertige Haarfärbemittel wird auf das Haar aufgetragen und mit einem Pinsel gleichmäßig verteilt. Nach einer Einwirkungszeit von 30 Minuten bei 40 °C wird das Haar mit einem Shampoo gewaschen, anschließend mit lauwarmem Wasser gespült und sodann getrocknet.

Das Haar kann zu jedem beliebigen Zeitpunkt (beispielsweise nach mehreren Tagen oder Wochen) mit einer 5 %igen Natriumsulfit-Lösung (pH = 5) innerhalb von 20 Minuten bei 40°C wieder vollständig entfärbt werden.
Die Färbe- und Entfärbeergebnisse sind in der nachfolgenden Tabelle 1 zusammengefasst.

Die in den vorliegenden Beispielen angegebenen L*a*b*-Farbmesswerte wurden mit einem Farbmessgerät der Firma Minolta, Typ Chromameter II, ermittelt. Hierbei steht der L-Wert für die Helligkeit (das heißt je geringer der L-Wert ist, umso größer ist die Farbintensität), während der a-Wert ein Maß für den Rotanteil ist (das heißt je größer der a-Wert ist, umso größer ist der Rotanteil). Der b-Wert ist ein Maß für den Blauanteil der Farbe, wobei der Blauanteil umso größer ist, je negativer der b-Wert ist.

Alle Prozentangaben in der vorliegenden Anmeldung stellen, sofern nicht anders angegeben, Gewichtsprozente dar.

**Tabelle 1: Färberesultate**

| **Nr.** | **in Komponente (A1) enthaltenes Enamin der Formeln (III) bis (X); in Komponente (A2) enthaltene Schiffsche Base der Formel (II)** | **Farbton nach der Färbung** | **Farbmeßwerte** | | | | **Farbton nach der Entfärbung** |
|---|---|---|---|---|---|---|---|
| | | | | **L** | **a** | **b** | |
| **2.1** | (A1) 1,2,3,3-Tetramethyl-3H-indolium-hydrogensulfat: 3,12 g, pH =1,7 | | unbehandelte Haare: | +83,30; | -0,48; | +10,40 | |
| | | intensiv rot | Nach dem Färben: | +32,50; | +54,44; | +17,61 | weiss |
| | (A2) 4-(((2-Hydroxyethyl)imino)-methyl)-2-methoxyphenol: 2,24 g und Monoethanolamin bis pH = 9,5 | | | | | | |
| **2.2** | (A1) 1,2,3,3-Tetramethyl-3H-indolium-chlorid: 2,42 g, pH = 1,7 (A2) 5-(((2-Hydroxyethyl)imino)-methyl)-2-methoxyphenol: 2,24 g und Monethanolamin bis pH 9,6 | intensiv gelb | unbehandelte Haare: | +83,30; | -0,48; | +10,40 | |
| | | | Nach dem Färben: | +67,73; | +24,42; | +83,08 | weiss |
| **2.3** | (A1) 1,2,3,3-Tetramethyl-3H-indolium-chlorid: 2,42 g, pH = 1,7 | | unbehandelte Haare: | +83,30; | -0,48; | +10,40 | |
| | | intensiv | Nach dem Färben: | +23,96; | +43,17; | -2,65 | weiss |
| | (A2) 2,6-Dimethoxy-4-(((2-hydroxyethyl)imino)methyl)phenol: 2,59 g und Monoethanolamin bis pH 9,6 | violett | | | | | |
| **2.4** | (A1) 1,2,3,3-Tetramethyl-3H- | | unbehandelte Haare: | +83,30; | -0,48; | +10,40 | |
| | indolium-hydrogensulfat: 3,13 g, | intensiv | Nach dem Färben: | +49,31; | +61,17; | +55,04 | weiss |
| | pH = 1,7 (A2) 4-(((2-Hydroxyethyl)imino)-methyl)phenol: 1,90 g und Monoethanolamin bis pH 9,4 | orange | | | | | |
| **2.5** | (A1) 1,2,3,3-Tetramethyl-3H-indolium- hydrogensulfat: 3,13 g, pH = 1,7 | | unbehandelte Haare: | +83,30; | -0,48; | +10,40 | |
| | | dunkel rot | Nach dem Färben: | +21,93; | +24,99; | +3,41 | weiss |
| | (A2) 1,2-Dihydroxy-4-(((2-hydroxyethyl)-imino)methyl)benzol: 2,08 g und Monoethanolamin bis pH 9,4 | | | | | | |
| **2.6** | (A1) 1,2,3,3-Tetramethyl-3H-indolium-hydrogensulfat: 3,12 g, | | unbehandelte Haare: | +83,30; | -0,48; | +10,40 | |
| | | intensiv | Nach dem Färben: | +39,83; | +67,33; | +10,32 | weiss |
| | pH = 1,7; | rosa-rot | | | | | |
| | (A2) N,N-dimethyl-4-(((2-hydroxyethyl)imino)methyl)anilin: 2,21 g ohne Monoethanolamin pH = 11,0 | | | | | | |
| **2.7** | (A1) 1,2,3,3-Tetramethyl-3H-indolium-hydrogensulfat: 3,12 g, pH =1,7; | | unbehandelte Haare: | +83,30; | -0,48; | +10,40 | |
| | | gelb-grün | Nach dem Färben: | +49,40; | +3,10; | +35,50 | weiss |
| | (A2) 1,2-Dihydroxy-3-(((2-Hydroxyethyl)imino)methyl)benzol: 2,08 g und Monoethanolamin bis pH = 9,6 | | | | | | |
| **2.8** | (A1) 1,2,3,3-Tetramethyl-3H-indolium-hydrogensulfat: 3,12 g, pH = 1,7; (A2) 4-(((3-Hydroxypropyl)-imino)methyl)phenol: 2,06 g und Monoethanolamin bis pH = 9,6 | | unbehandelte Haare: | +83,30; | -0,48; | +10,40 | |
| | | intensiv orange | Nach dem Färben: | +49,99; | +60,78; | +53,05 | weiss |
| **2.9** | (A1) 1,2,3,3-Tetramethyl-3H-indolium-hydrogensulfat: 3,12 g, pH = 1,7; (A2) 2,6-Dimethoxy-4-(((3-hydroxypropyl)imino)methyt)phenol: 2,75 g und Monoethanolamin bis pH = 9,7 | | unbehandelte Haare: | +83,30; | -0,48; | +10,40 | |
| | | intensiv violett | Nach dem Färben: | +20,87; | +42,77; | -1,23 | weiss |
| **2.10** | (A1) 1,2,3,3-Tetramethyl-3H-indolium-hydrogensulfat: 3,12 g, pH = 1,7; (A2) 4-(((2,3-Dihydroxypropyl)imino)methyl)phenol: 2,25 g und Monoethanolamin bis pH = 9,6 | | unbehandelte Haare: | +83,30; | -0,48; | +10,40 | |
| | | intensiv orange | Nach dem Färben: | +50,66; | +58,05; | +55,55 | weiss |
| **2.11** | (A1) 1,2,3,3-Tetramethyl-3H-indolium-hydrogensulfat: 3,12 g, pH =1,7; (A2) 2,6-Dimethoxy-4-(((2,3-dihydroxypropyl)imino)methyl)phenol: 2,94 g und Monoethanolamin bis pH = 9,4 | | unbehandelte Haare: | +83,30; | -0,48; | +10,40 | |
| | | intensiv violett | Nach dem Färben: | +21,18; | +40,69; | -2,99 | weiss |
| **2.12** | (A1) 1,2,3,3-Tetramethyl-3H-indolium-hydrogensulfat: 3,12 g, pH = 1,7; | | unbehandelte Haare: | +83,30; | -0,48; | +10,40 | |
| | | intensiv orange | Nach dem Färben: | +50,85; | +56,67; | +54,97 | weiss |
| | (A2) 2-[(4-Hydroxy-benzyliden)-amino]-propan-1,3-diol: 2,25 g und Monoethanolamin bis pH = 9,7 | | | | | | |
| **2.13** | (A1) 1,2,3,3-Tetramethyl-3H-indolium-hydrogensulfat: 3,12 g, pH =1,7; (A2) 2-[(4-Hydroxy-3,5-dimethoxy-benzyliden)-amino]-propan-1,3-diol: 2,94 g und Monoethanolamin bis pH = 9,2 | | unbehandelte Haare: | +83,30; | -0,48; | +10,40 | |
| | | intensiv violett | Nach dem Färben: | +22,21; | +42,35; | -1,60 | weiss |
| **2.14** | (A1) 1,2,3,3-Tetramethyl-3H-indolium-hydrogensulfat: 3,12 g, pH =1,7; | | unbehandelte Haare: | +83,30; | -0,48; | +10,40 | |
| | | intensiv orange | Nach dem Färben: | +51,66; | +55,67; | +55,08 | weiss |
| | (A2) 4-(((2-Hydroxy-2-phenyl-ethyl)imino)methyl)phenol: 2,77 g und Monoethanolamin bis pH = 9,6 | | | | | | |
| **2.15** | (A1) 1,2,3,3-Tetramethyl-3H-indolium-hydrogensulfat: 3,12 g, pH = 1,7; (A2) 2,6-Dimethoxy-4-(((1-phenyl-2-hydroxyethyl)imino)methyl)phenol: 3,47 g und Monoethanolamin bis pH = 9,5 | | unbehandelte Haare: | +83,30; | -0,48; | +10,40 | |
| | | intensiv violett | Nach dem Färben: | +22.07; | +39,20; | +0,32 | weiss |
| **2.16** | (A1) 1,2,3,3-Tetramethyl-3H-indolium-hydrogensulfat: 3,12 g, pH = 1,7; (A2) 4-(((2-Hydroxyphenyl)-imino)methyl)phenol: 2,45 g und Monoethanolamin bis pH = 8,9 | | unbehandelte Haare: | +83,30; | -0,48; | +10,40 | |
| | | intensiv orange | Nach dem Färben: | +49,11; | +56,69; | +54,91 | weiss |
| **2.17** | (A1) 1,2,3,3-Tetramethyl-3H-indolium-hydrogensulfat: 3,12 g, pH = 1,7; (A2) 2,6-Dimethoxy-4-(((2-hydroxyphenyl)imino)methyl)phenol: 3,14 g und Monoethanolamin bis pH = 9,5 | | unbehandelte Haare: | +83,30; | -0,48; | +10,40 | |
| | | intensiv violett | Nach dem Färben: | +20,95; | +34,57; | +0,68 | weiss |
| **2.18** | (A1) 1,2,3,3-Tetramethyl-3H-indolium-hydrogensulfat: 3,12 g, pH = 1,7 (A2) 5-Guanidino-2-[(4-hydroxy-benzyliden)-amino]-pentansäure: 3,20 g und Monoethanolamin bis pH = 9,5 | | unbehandelte Haare: | +83,30; | -0,48; | +10,40 | |
| | | intensiv orange | Nach dem Färben: | +52,97; | +55,56; | +58,45 | weiss |
| **2.19** | (A1) 1,2,3,3-Tetramethyl-3H-indolium-hydrogensulfat: 3,12 g, pH = 1,7 (A2) 2-[(4-Dimethylamino-naphthalen-1-ylmethylen)-amino]-ethanol: 2,79 g und Monoethanolamin bis pH = 11,0 | | unbehandelte Haare: | +83,30; | -0,48; | +10,40 | |
| | | intensiv violett | Nach dem Färben: | +35,40; | +20,58; | -15,69 | weiss |
| **2.20** | (A1) 5-Hydroxy-1,2,3,3-Tetramethyl- 3H-indolium-jodid: 3,65 g, pH = 3,7 (A2) 2-[(4-Dimethylamino-naphthalen-1-ylmethylen)-amino]-ethanol: 2,79 g und Monoethanolamin bis pH = 11.0 | | unbehandelte Haare: | +83,30; | -0,48; | +10,40 | |
| | | intensiv violett | Nach dem Färben: | 36,34; | 12,41 ; | -8,50 | weiss |

## Patentansprüche

1. Mittel zur Färbung von Fasern (A), welches durch Vermischen zweier Komponenten (A1) und (A2) hergestellt wird und **dadurch gekennzeichnet ist, dass** die Komponente (A1) einen sauren pH-Wert aufweist und mindestens ein Enamin der Formel (III) bis (X) oder dessen Säureadditionssalz enthält, 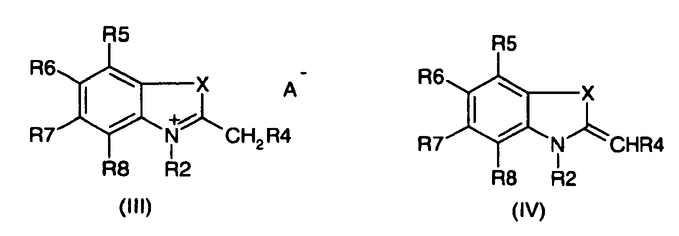 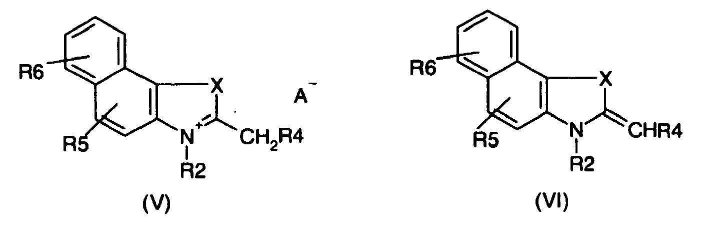 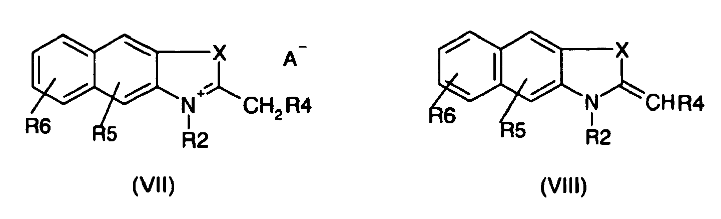 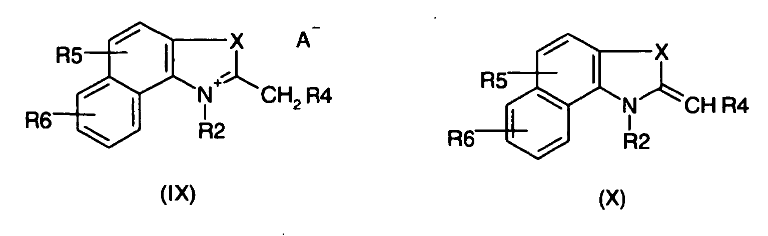 in denen **X** gleich einem mit zwei C1- bis C4-Alkylgruppen, welche gleich oder verschieden sein können, substituierten C-Atom, einem mit einer C1- bis C4-Alkylgruppe und einer Hydroxygruppe substituierten C-Atom, einem Schwefelatom, einem alkylierten Stickstoffatom, einem nichtalkylierten Stickstoffatom oder einem Sauerstoffatom ist; und **R2** gleich einer geradkettigen oder verzweigten C1- bis C8-Alkylgruppe, einer geradkettigen oder verzweigten C1- bis C8-Hydroxyalkylgruppe, oder einer C1- bis C8-Alkoxyalkylgruppe ist, wobei jeweils zwischen den C-Atomen der Alkylkette Sauerstoffatome sitzen können; **R4** gleich Wasserstoff, einer geradkettigen C1- bis C4-Alkylgruppe oder einer verzweigten C1- bis C4-Alkylgruppe ist; **R5, R6, R7** und **R8** unabhängig voneinander gleich Wasserstoff, einer geradkettigen oder verzweigten C1- bis C4-Alkylgruppe, einer geradkettigen oder verzweigten C1-bis C4-Hydroxyalkylgruppe, einer Hydroxygruppe, einer Methoxygruppe, einer Aminogruppe, einer Monoalkylaminogruppe, einer Dialkylaminogruppe, einer Benzylgruppe oder einem Halogenatom sind; **R9** gleich Wasserstoff, einer geradkettigen C1-bis C4-Alkylgruppe oder einer verzweigten C1- bis C4-Alkylgruppe ist; und **A⁻** gleich Chlorid, Bromid, Jodid, Sulfat, Hydrogensulfat, Toluolsulfonat, Benzolsulfonat, Monomethylsulfat, Hexafluorphosphat, Hexafluorantimonat, Tetrafluorborat, Tetraphenylborat, Formiat, Acetat oder Propionat ist;
und die Komponente (A2) einen alkalischen pH-Wert aufweist und mindestens eine Schiffsche Base der Formel (II) enthält,
R₇HC=NR₈ (II)
wobei gilt: **R7** ist gleich einer Restgruppe der Formeln 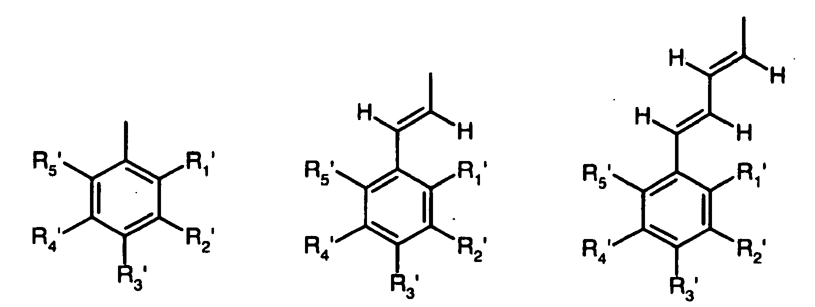 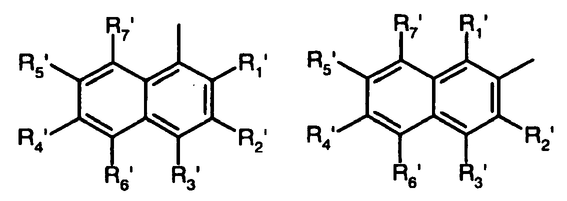 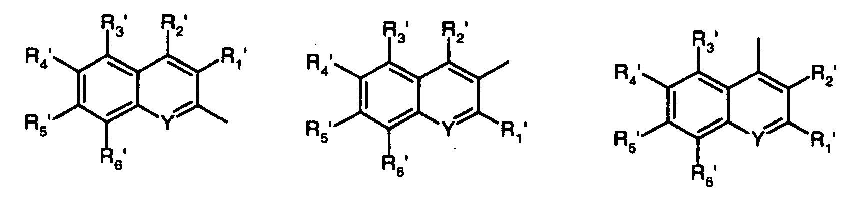 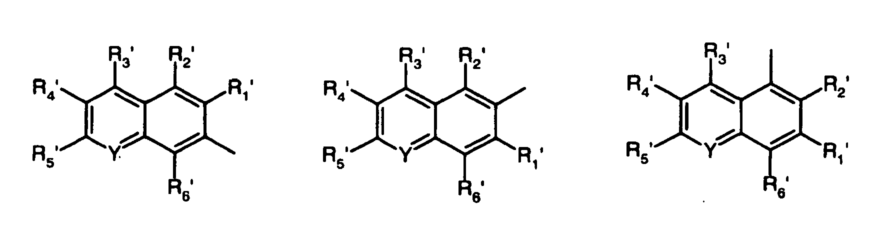 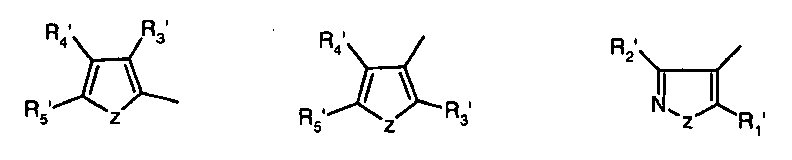 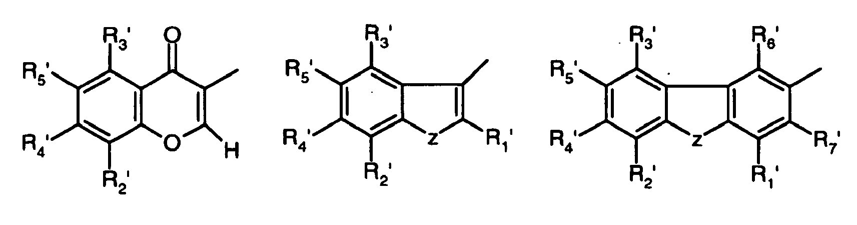 wobei **-Y=** gleich -N=, -N⁺R^{a}=, -O⁺= oder -S⁺= ist, und **Z** gleich einem Sauerstoffatom, einem Schwefelatom oder einer NR^{a}-Gruppe ist; **R1', R2', R3', R4', R5', R6'** und **R7'** unabhängig voneinander gleich einem Wasserstoffatom, einer Hydroxygruppe, einer Methoxygruppe, einer Arylgruppe, einem Halogenatom, einer -CHO-Gruppe, einer -COR^{a} -Gruppe, einer -CO₂R^{a} -Gruppe, einer NO₂-Gruppe, einer -OCOR^{a} -Gruppe, einer -OCH₂Aryl-Gruppe, einer -NH₂ -Gruppe, einer -NH₃⁺-Gruppe, einer -NHR^{a} -Gruppe, einer -NR^{a}H₂⁺-Gruppe, einer-N(R^{a})₂-Gruppe, einer -N(R^{a})₃⁺-Gruppe, einer -NHCOR^{a} -Gruppe, einer-NHCOOR^{a} -Gruppe, mit R^{a} gleich einem Wasserstoffatom, einer geradkettigen oder verzweigten C1 bis C4-Alkylgruppe, einem gegebenenfalls substituierten aromatischen Carbozyklus oder Heterocyclus, sind; unter der Bedingung, dass mindestens einer der Reste R1' bis R7' von Wasserstoff verschieden ist; und **R8** gleich einer Restgruppe der Formeln, 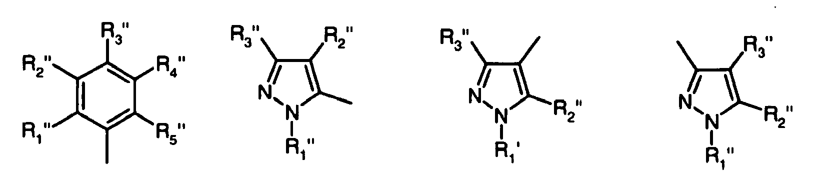 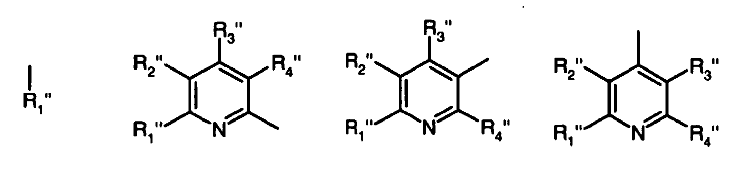 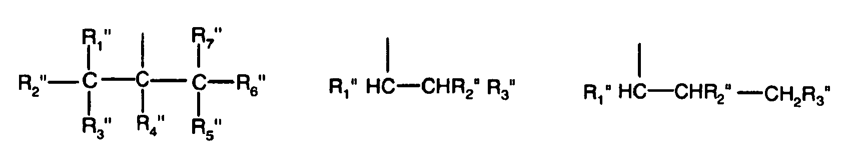 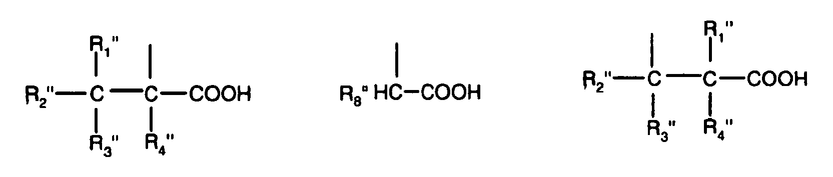 wobei **R1", R2", R3", R4", R5", R6"** und **R7"** unabhängig voneinander gleich einem Wasserstoffatom, einer Methylgruppe, einem Halogenatom, einer Hydroxygruppe, einer C1- bis C4-Hydroxyalkylgruppe, einer Benzylgruppe, einem gegebenenfalls substituierten aromatischen Carbozyklus oder Heterocyclus, einer Methoxygruppe, einer Ethoxy-gruppe, einer Carboxygruppe, einer -NH₂-Gruppe, einer -NHR^{b}-Gruppe, einer-N(R^{b})₂-Gruppe sind, mit R^{b} gleich einem Wasserstoffatom, einer geradkettigen oder verzweigten C1 bis C4-Alkylgruppe, einer C1- bis C4- Hydroxyalkylgruppe, einem gegebenenfalls substituierten aromatischen Carbozyklus oder einem gegebenenfalls substituierten aromatischen Heterocyclus; und **R8"** gleich einem zur Bildung einer natürlichen α-Aminosäure erforderlichen Restes ist.

2. Mittel nach Anspruch 1, **dadurch gekennzeichnet, dass** die Verbindung der Formeln (III) bis (X) ausgewählt ist aus 1,3,3-Trimethyl-2-methylen-indolin sowie dessen Salzen, 1,3,3,4-Tetramethyl-2-methylen-indolin sowie dessen Salzen, 1,3,3,5-Tetramethyl-2-methylen-indolin sowie dessen Salzen, 1,3,3,6-Tetramethyl-2-methylen-indolin sowie dessen Salzen, 1,3,3,7-Tetramethyl-2-methylen-indolin sowie dessen Salzen, 1,3,3,6,7-Pentamethyl-2-methylen-indolin sowie dessen Salzen, 1,3,3,5,7-Pentamethyl-2-methylen-indolin sowie dessen Salzen, 1,3,3,4,7-Pentamethyl-2-methylen-indolin sowie dessen Salzen, 5-Chloro-1,3,3-trimethyl-2-methylen-indolin sowie dessen Salzen, 5-Fluoro-1,3,3-trimethyl-2-methylen-indolin sowie dessen Salzen, 5-Isopropyl-1,3,3-trimethyl-2-methylen-indolin sowie dessen Salzen, 5-Hydroxy-1,3,3-trimethyl-2-methylen-indolin sowie dessen Salzen, 5-Methoxy-1,3,3-trimethyl-2-methylen-indolin sowie dessen Salzen, 5-Amino-1,3,3-trimethyl-2-methylen-indolin sowie dessen Salzen, 5-N-acetylamino-1,3,3-trimethyl-2-methylen-indolin sowie dessen Salzen, 5-Methoxy-6-N-acetylamino-1,3,3-trimethyl-2-methylen-indolin sowie dessen Salzen, 5-Hydroxy-7-N-acetylamino-1,3,3-trimethyl-2-methylen-indolin sowie dessen Salzen, 6-Hydroxy-1,3,3-trimethyl-2-methylen-indolin sowie dessen Salzen, 6-Methoxy-1,3,3-trimethyl-2-methylen-indolin sowie dessen Salzen, 5-Methoxy-6-amino-1,3,3-trimethyl-2-methylen-indolin-sowie dessen Salzen, 5,6-Dihydroxy-1,3,3-trimethyl-2-methylen-indolin sowie dessen Salzen, 5,6-Dimethoxy-1,3,3-trimethyl-2-methylen-indolin sowie dessen Salzen, 4,5-Dihydroxy-1,3,3-trimethyl-2-methylen-indolin sowie dessen Salzen, 5,7-Dihydroxy-1,3,3-trimethyl-2-methylen-indolin sowie dessen Salzen, 5-Amino-6-methoxy-1,3,3-trimethyl-2-methylen-indolin sowie dessen Salzen, 5-Amino-7-hydroxy-1,3,3-trimethyl-2-methylen-indolin sowie dessen Salzen, 5-Hydroxy-7-amino-1,3,3-trimethyl-2-methylen-indolin sowie dessen Salzen, 1-(2'-Hydroxyethyl)-3,3-dimethyl-2-methylen-indolin sowie dessen Salzen, 1,3,3-Trimethyl-2-methylen-3H-benz[e]indol sowie dessen Salzen und N-Ethyl-2-methylen-benzthiazol sowie dessen Salzen.

3. Mittel nach Anspruch 1 oder 2, **dadurch gekennzeichnet, dass** die Schiffsche Base der Formel (II) ausgewählt ist aus 4-(((2-Hydroxyethyl)imino)methyl)-2-methoxyphenol, 5-(((2-Hydroxyethyl)-imino)methyl)-2-methoxyphenol, 2,6-Dimethoxy-4-(((2-hydroxyethyl)-imino)methyl)phenol, 4-(((2-Hydroxyethyl)imino)-methyl)phenol, 1,2-Dihydroxy-4-(((2-hydroxyethyl)imino)-methyl)benzol, N,N-dimethyl-4-(((2-hydroxyethyl)imino)methyl)-anilin, 1,2-Dihydroxy-3-(((2-hydroxyethyl)-imino)methyl)benzol, 4-(((3-Hydroxypropyl)imino)methyl)phenol, 2,6-Dimethoxy-4-(((3-Hydroxypropyl)imino)methyl)phenol, 4-(((2,3-Dihydroxypropyl)imino)-methyl)phenol, 2,6-Dimethoxy-4-(((2,3-dihydroxypropyl)imino)-methyl)phenol, 2-[(4-Hydroxy-benzyliden)-amino]-propan-1,3-diol, 2-[(4-Hydroxy-3,5-dimethoxy-benzyliden)-amino]-propan-1,3-diol, 4-(((2-Hydroxy-2-phenyl-ethyl)imino)methyl)phenol, 2,6-Dimethoxy-4-(((1-phenyl-2-hydroxy-ethyl)imino)methyl)phenol, 4-(((2-hydroxyphenyl)imino)methyl)phenol, 2,6-Dimethoxy-4-(((2-hydroxyphenyl)imino)methyl)phenol, 5-Guanidino-2-[(4-hydroxy-benzyliden)-amino]-pentansäure, 2-[(4-Dimethylamino-naphthalen-1-ylmethylen)-amino]-ethanol, 5-Guanidino-2-[(4-hydroxy-3,5-dimethoxy-benzyliden)-amino]-pentansäure, 2-[(4-Hydroxy-3,5-dimethoxy-benzyliden)-amino]-3-(3*H-*imidazol-4-yl)-propansäure, 2-[(4-Hydroxy-benzyliden)-amino]-3-(3*H-*imidazol-4-yl)-propansäure, 2-[(4-Hydroxy-3,5-dimethoxy-benzyliden)-amino]-3-(1*H*-indol-3-yl)-propansäure, 2-[(4-Hydroxy-benzyliden)-amino]-3-(1*H*-indol-3-yl)-propansäure, 2-(((2-Hydroxyethyl)imino)methyl)pheno), 1,2-Dihydroxy-3-(((2-hydroxyethyl)imino)methyl)benzol, 1,2,3-Trihydroxy-4-(((2-hydroxy-ethyl)imino)methyl)benzol, 1,2,3,4-Tetrahydroxy-5-(((2-hydroxyethyl)-imino)methyl)benzol und 1,2,4-Trihydroxy-4-(((2-hydroxyethyl)imino)-methyl)benzol.

4. Mittel nach einem der Ansprüche 1 bis 3, **dadurch gekennzeichnet, dass** die Komponente (A1) einen pH-Wert von 1 bis 4,5 hat.

5. Mittel nach einem der Ansprüche 1 bis 4, **dadurch gekennzeichnet, dass** die Komponente (A2) einen pH-Wert von 7,5 bis 12 hat.

6. Mittel nach einem der Ansprüche 1 bis 5, **dadurch gekennzeichnet, dass** es das Enamin der Formel (III) bis (X) sowie die Schiffsche Base der Formel (II), bezogen auf die gebrauchsfertige Zubereitung, jeweils in einer Gesamtmenge von 0,01 bis 10 Gewichtsprozent enthält.

7. Mittel nach einem der Ansprüche 1 bis 6, **dadurch gekennzeichnet, dass** die Komponenten (A1) und (A2) in Form einer Lösung, einer Emulsion, eines Schaumes, einer Creme, eines Gels, eines Pulvers, eines Granulats oder einer Brausetablette vorliegen.

8. Verfahren zur temporären Färbung von Fasern, insbesondere Haaren, bei dem die Faser mit einem Mittel nach einem der Ansprüche 1 bis 7 gefärbt wird und zu einem beliebigen späteren Zeitpunkt wieder entfärbt wird, **dadurch gekennzeichnet, dass** man zur Entfärbung eine sulfithaltige Zubereitung für eine Dauer von 5 bis 60 Minuten bei einer Temperatur von 20 bis 50 °C auf die gefärbte Faser einwirken läßt.

9. Mehrkomponenten-Kit, bestehend aus einer ein Enamin der Formel (III) bis (X) gemäß Anspruch 1 enthaltenden Komponente (A1) und einer eine Schiffsche Base der Formel (II) gemäß Anspruch 1 enthaltenden Komponente (A2).

## Claims

1. Agent for colouring fibres (A) which is prepared by mixing two components (A1) and (A2) and is **characterized in that** component (A1) has an acidic pH and comprises at least one enamine of the formula (III) to (X) or acid addition salt thereof, 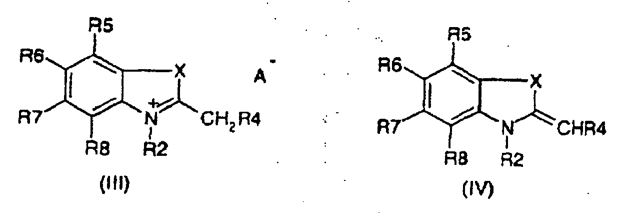 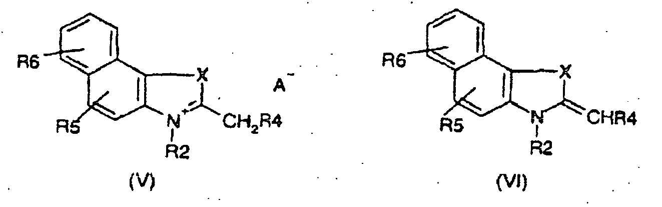 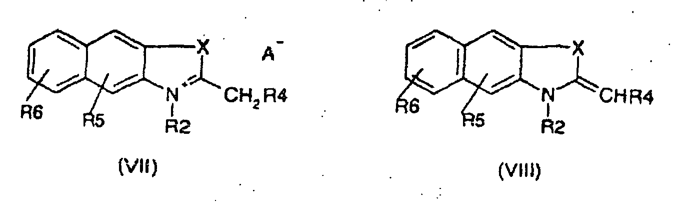 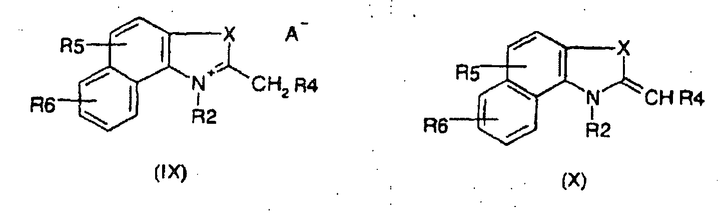 in which **X** is a carbon atom substituted by two C1-to C4-alkyl groups, which may be identical or different, a carbon atom substituted by a C1- to C4-alkyl group and a hydroxy group, a sulphur atom, an alkylated nitrogen atom, a nonalkylated nitrogen atom or an oxygen atom; and **R2** is a straight-chain or branched C1- to C8-alkyl group, a straight-chain or branched C1- to C8-hydroxyalkyl group, or a C1- to C8-alkoxyalkyl group, where in each case oxygen atoms can sit between the carbon atoms of the alkyl chain; **R4** is hydrogen, a straight-chain C1- to C4-alkyl group or a branched C1- to C4-alkyl group; **R5, R6, R7** and **R8,** independently of one another, are hydrogen, a straight-chain or branched C1- to C4-alkyl group, a straight-chain or branched C1-to C4-hydroxyalkyl group, a hydroxy group, a methoxy group, an amino group, a monoalkylamino group, a dialkylamino group, a benzyl group or a halogen atom; **R9** is hydrogen, a straight-chain C1-to C4-alkyl group or a branched C1- to C4-alkyl group; and **A-** is chloride, bromide, iodide, sulphate, hydrogensulphate, toluenesulphonate, benzenesulphonate, monomethylsulphate, hexafluorophosphate, hexafluoroantimonate, tetrafluoroborate, tetraphenylborate, formate, acetate or propionate;
and component (A2) has an alkaline pH and comprises at least one Schiff's base of the formula (II),
R₇HC=NR₈ (II)
where: **R7** is a radical group of the formulae 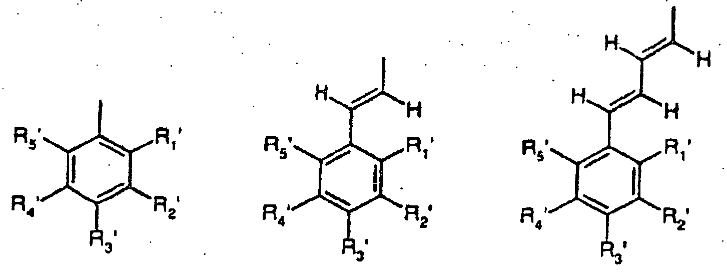 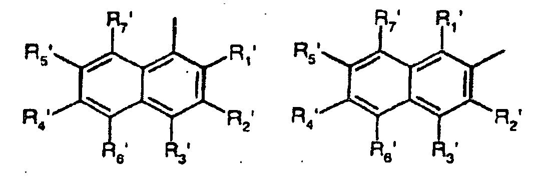 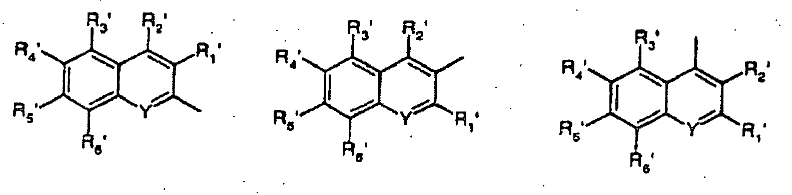 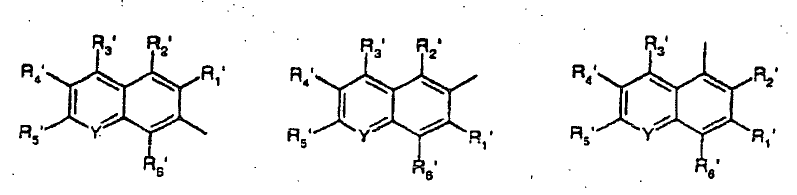 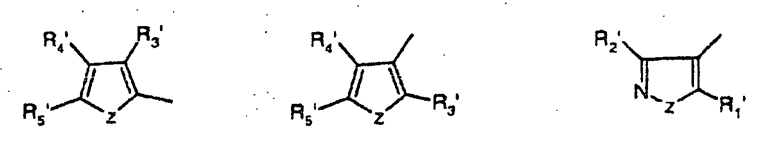 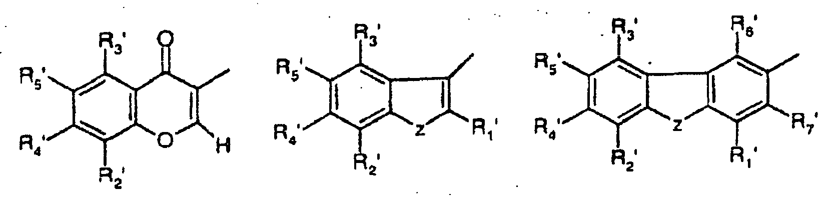 where **-Y=** is -N=, -N⁺R^{a}=, -O⁺= or -S⁺=, and **Z** is an oxygen atom, a sulphur atom or an NR^{a} group; **R1', R2', R3', R4', R5', R6'** and **R7',** independently of one another, are a hydrogen atom, a hydroxy group, a methoxy group, an aryl group, a halogen atom, a -CHO group, a -COR^{a} group, a -CO₂R^{a} group, an NO₂ group, an -OCOR^{a} group, an -OCH₂aryl group, an -NH₂ group, an -NH₃⁺ group, an -NHR^{a} group, an -NR^{a}H₂⁺ group, an -N(R^{a})₂ group, an -N(R^{a})₃⁺ group, an -NHCOR^{a} group, an -NHCOOR^{a} group, where R^{a} is a hydrogen atom, a straight-chain or branched C1 to C4-alkyl group, an optionally substituted aromatic carbocycle or heterocycle; with the proviso that at least one of the radicals R1' to R7' is different from hydrogen; and **R8** is a radical group of the formulae, 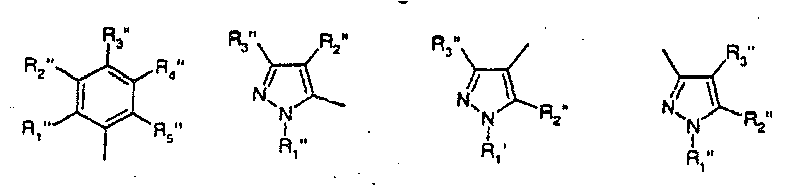 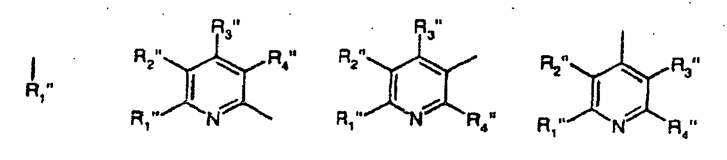 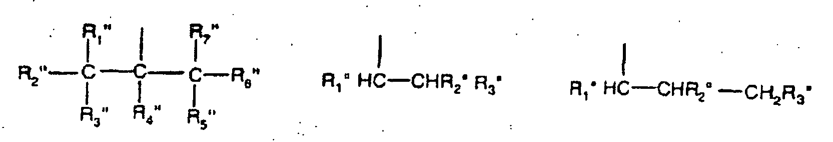 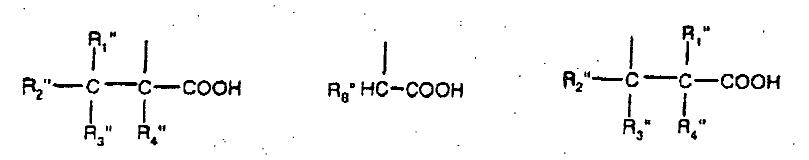 where **R1", R2", R3", R4", R5", R6"** and **R7",** independently of one another, are a hydrogen atom, a methyl group, a halogen atom, a hydroxy group, a C1- to C4-hydroxyalkyl group, a benzyl group, an optionally substituted aromatic carbocycle or heterocycle, a methoxy group, an ethoxy group, a carboxy group, an -NH₂ group, an -NHR^{b} group, an -N(R^{b})₂ group, where R^{b} is a hydrogen atom, a straight-chain or branched C1 to C4-alkyl group, a C1- to C4-hydroxyalkyl group, an optionally substituted aromatic carbocycle or an optionally substituted aromatic heterocycle; and **R8"** is a radical required to form a natural a-amino acid.

2. Agent according to Claim 1, **characterized in that** the compound of the formulae (III) to (X) is selected from 1,3,3-trimethyl-2-methyleneindoline and salts thereof, 1,3,3,4-tetramethyl-2-methyleneindoline and salts thereof, 1,3,3,5-tetramethyl-2-methyleneindoline and salts thereof, 1,3,3,6-tetramethyl-2-methyleneindoline and salts thereof, 1,3,3,7-tetramethyl-2-methyleneindoline and salts thereof, 1,3,3,6,7-pentamethyl-2-methyleneindoline and salts thereof, 1,3,3,5,7-pentamethyl-2-methyleneindoline and salts thereof, 1,3,3,4,7-pentamethyl-2-methyleneindoline and salts thereof, 5-chloro-1,3,3-trimethyl-2-methyleneindoline and salts thereof, 5-fluoro-1,3,3-trimethyl-2-methyleneindoline and salts thereof, 5-isopropyl-1,3,3-trimethyl-2-methyleneindoline and salts thereof, 5-hydroxy-1,3,3-trimethyl-2-methyleneindoline and salts thereof, 5-methoxy-1,3,3-trimethyl-2-methyleneindoline and salts thereof, 5-amino-1,3,3-trimethyl-2-methyleneindoline and salts thereof, 5-N-acetylamino-1,3,3-trimethyl-2-methyleneindoline and salts thereof, 5-methoxy-6-N-acetylamino-1,3,3-trimethyl-2-methyleneindoline and salts thereof, 5-hydroxy-7-N-acetylamino-1,3,3-trimethyl-2-methyleneindoline and salts thereof, 6-hydroxy-1,3,3-trimethyl-2-methyleneindoline and salts thereof, 6-methoxy-1,3,3-trimethyl-2-methyleneindoline and salts thereof, 5-methoxy-6-amino-1,3,3-trimethyl-2-methyleneindoline and salts thereof, 5,6-dihyroxy-1,3,3-trimethyl-2-methyleneindoline and salts thereof, 5,6-dimethoxy-1,3,3-trimethyl-2-methyleneindoline and salts thereof, 4,5-dihyroxy-1,3,3-trimethyl-2-methyleneindoline and salts thereof, 5,7-dihyroxy-1,3,3-trimethyl-2-methyleneindoline and salts thereof, 5-amino-6-methoxy-1,3,3-trimethyl-2-methyleneindoline and salts thereof, 5-amino-7-hydroxy-1,3,3-trimethyl-2-methyleneindoline and salts thereof, 5-hydroxy-7-amino-1,3,3-trimethyl-2-methyleneindoline and salts thereof, 1-(2'-ydroxyethyl)-3,3-dimethyl-2-methyleneindoline and salts thereof, 1,3,3-trimethyl-2-methylene-3H-benz[e]indole and salts thereof and N-ethyl-2-methylenebenzothiazole and salts thereof.

3. Agent according to Claim 1 or 2, **characterized in that** the Schiff's base of the formula (II) is selected from 4-(((2-hydroxyethyl)imino)methyl)-2-methoxyphenol, 5-(((2-hydroxyethyl)imino)-methyl)-2-methoxyphenol, 2,6-dimethoxy-4-(((2-hydroxyethyl)imino)methyl)phenol, 4-(((2-hydroxyethyl)imino)methyl)phenol, 1,2-dihydroxy-4-(((2-hydroxyethyl)imino)methyl)-benzene, N,N-dimethyl-4-(((2-hydroxyethyl)imino)-methyl)aniline, 1,2-dihydroxy-3-(((2-hydroxyethyl)imino)methyl)benzene, 4-(((3-hydroxypropyl)-imino)methyl)phenol, 2,6-dimethoxy-4-(((3-hydroxypropyl)imino)methyl)phenol, 4-(((2,3-dihydroxypropyl)imino)methyl)phenol, 2,6-dimethoxy-4-(((2,3-dihydroxypropyl)imino)methyl)phenol, 2-[(4-hydroxybenzylidene)amino]propane-1,3-diol, 2-[(4-hydroxy-3,5-dimethoxybenzylidene)amino]-propane-1,3-diol, 4-(((2-hydroxy-2-phenylethyl)-imino)methyl)phenol, 2,6-dimethoxy-4-(((1-phenyl-2-hydroxyethyl)imino)methyl)phenol, 4-(((2-hydroxyphenyl)imino)methyl)phenol, 2,6-dimethoxy-4-(((2-hydroxyphenyl)imino)methyl)-phenol, 5-guanidino-2-[(4-hydroxybenzylidene)-amino]pentanoic acid, 2-[(4-dimethylaminonaphthalen-1-ylmethylene)amino]ethanol, 5-guanidino-2-[(4-hydroxy-3,5-dimethoxybenzylidene)amino]-pentanoic acid, 2-[(4-hydroxy-3,5-dimethoxybenzylidene)amino]-3-(3H-imidazol-4-yl)propanoic acid, 2-[(4-hydroxybenzylidene)amino]-3-(3H-imidazol-4-yl)propanoic acid, 2-[(4-hydroxy-3,5-dimethoxybenzylidene)amino]-3-(1H-indol-3-yl)propanoic acid, 2-[(4-hydroxybenzylidene)-amino]-3-(1H-indol-3-yl)propanoic acid, 2-(((2-hydroxyethyl)imino)methyl)phenol, 1,2-dihydroxy-3-(((2-hydroxyethyl)imino)methyl)-benzene, 1,2,3-trihydroxy-4-(((2-hydroxyethyl)-imino)methyl)benzene, 1,2,3,4-tetrahydroxy-5-(((2-hydroxyethyl)imino)methyl)benzene and 1,2,4-trihydroxy-4-(((2-hydroxyethyl)imino)-methyl)benzene.

4. Agent according to one of Claims 1 to 3, **characterized in that** component (A1) has a pH of from 1 to 4.5.

5. Agent according to one of Claims 1 to 4, **characterized in that** component (A2) has a pH of from 7.5 to 12.

6. Agent according to one of Claims 1 to 5, **characterized in that** it comprises the enamine of the formula (III) to (X) and the Schiff's base of the formula (II), based on the ready-to-use preparation, in each case in a total amount of from 0.01 to 10 per cent by weight.

7. Agent according to one of Claims 1 to 6, **characterized in that** components (A1) and (A2) are present in the from of a solution, an emulsion, a foam, a cream, a gel, a powder, granules or an effervescent tablet.

8. Method for temporarily colouring fibres, in particular hair, in which the fibre is coloured with an agent according to one of Claims 1 to 7 and is decoloured again at any desired subsequent time point, **characterized in that**, for the decolouring, a sulphite-containing preparation is left to act on the coloured fibre for a period of from 5 to 60 minutes at a temperature of from 20 to 50°C.

9. Multicomponent kit consisting of a component (A1) comprising an enamine of the formula (III) to (X) according to Claim 1, and a component (A2) comprising a Schiff's base of the formula (II) according to Claim 1.

## Revendications

1. Composition pour la teinture de fibres (A), qui est préparée par mélange de deux composants (A1) et (A2) et est **caractérisée en ce que** le composant (A1) présente un pH acide et contient au moins une énamine de formule (III) à (X) ou un sel d'addition de celle-ci avec un acide, 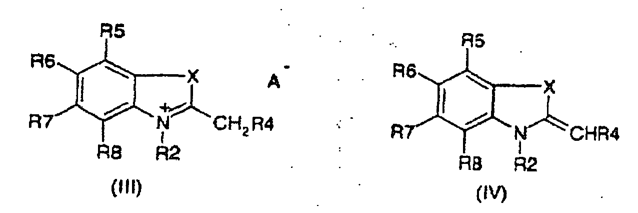 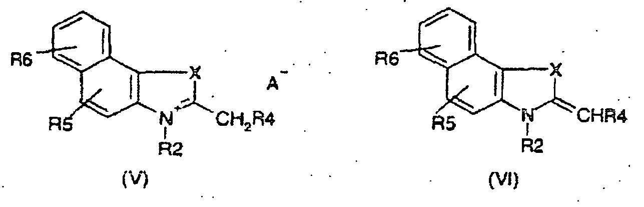 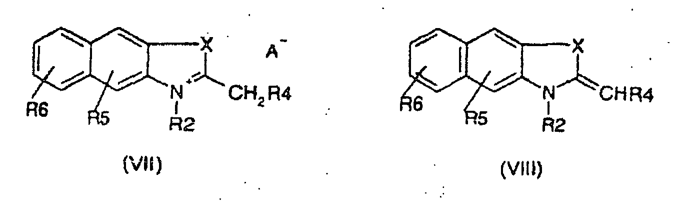 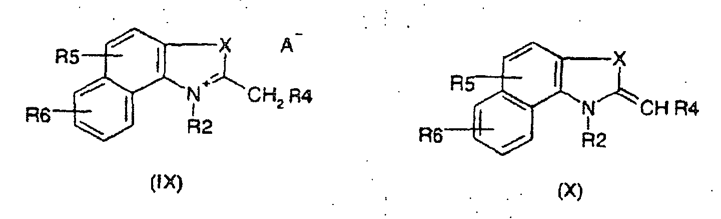 formules dans lesquelles **X** représente un atome de carbone substitué par deux groupes alkyle en C₁-C₄ qui peuvent être identiques ou différents, un atome de carbone substitué par un groupe alkyle en C₁-C₄ et un groupe hydroxy, un atome de soufre, un atome d'azote alkylé, un atome d'azote non alkylé ou un atome d'oxygène ; et **R2** représente un groupe alkyle en C₁-C₈ à chaîne droite ou ramifiée, un groupe hydroxyalkyle en C₁-C₈ à chaîne droite ou ramifiée ou un groupe alcoxyalkyle en C₁-C₈, des atomes d'oxygène pouvant dans chaque cas se trouver entre les atomes de carbone de la chaîne alkyle ; **R4** représente un atome d'hydrogène, un groupe alkyle en C₁-C₄ à chaîne droite ou un groupe alcoxy en C₁-C₄ ramifié ; **R5, R6, R7** et **R8** représentent, indépendamment les uns des autres, un atome d'hydrogène, un groupe alkyle en C₁-C₄ à chaîne droite ou ramifiée, un groupe hydroxyalkyle en C₁-C₄ à chaîne droite ou ramifiée, un groupe hydroxy, un groupe méthoxy, un groupe amino, un groupe monoalkylamino, un groupe dialkylamino, un groupe benzyle ou un atome d'halogène ; **R9** représente un atome d'hydrogène, un groupe alkyle en C₁-C₄ à chaîne droite ou un groupe alkyle en C₁-C₄ ramifié ; et **A⁻** représente l'anion chlorure, bromure, iodure, sulfate, hydrogénosulfate, toluènesulfonate, benzènesulfonate, monométhylsulfate, hexafluorophosphate, hexafluoroantimonate, tétrafluoroborate, tétraphénylborate, formiate, acétate ou propionate ;
et le composant (A2) présente un pH alcalin et contient au moins une base de Schiff de formule (II),
R-₇HC=NR₈ (II)
où : **R7** est un groupe de formules 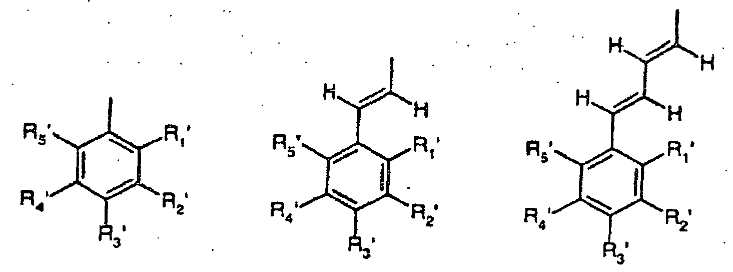 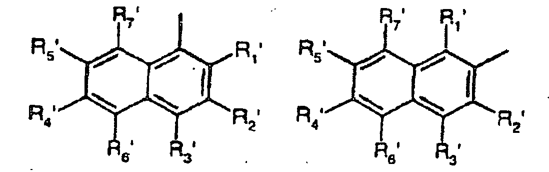 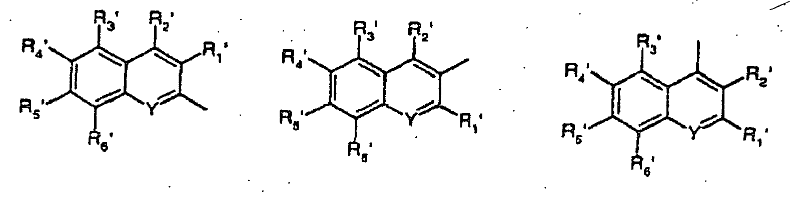 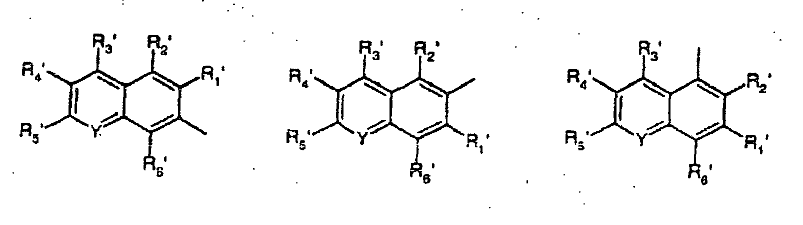 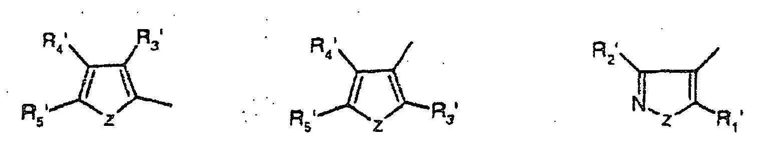 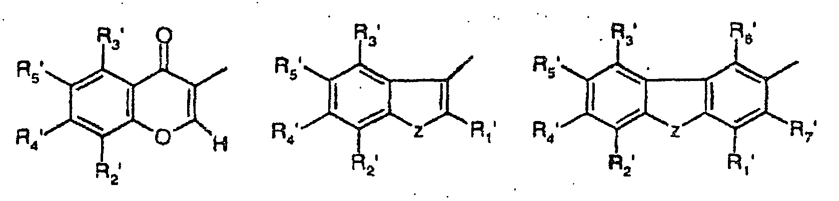 dans lesquelles **-Y= représente** -N=, -N⁺R^{a}=, -O⁺= ou -S⁺=, et **Z** est un atome d'oxygène, un atome de soufre ou un groupe NR^{a} ; **R1', R2', R3', R4', R5', R6'** et **R7'** représentent, indépendamment les uns des autres, un atome d'hydrogène, un groupe hydroxy, un groupe méthoxy, un groupe aryle, un atome d'halogène, un groupe -CHO, un groupe -COR^{a}, un groupe -CO₂R^{a}, un groupe NO₂, un groupe -OCOR^{a}, un groupe -OCH₂-aryle, un groupe -NH₂, un groupe -NH₃⁺, un groupe -NHR^{a}, un groupe -NR^{a}H₂⁺, un groupe -N(R^{a})₂, un groupe -N(R^{a})₃⁺, un groupe -HCOR^{a}, un groupe -NHCOOR^{a}, où R^{a} représente un atome d'hydrogène, un groupe alkyle en C₁-C₄ à chaîne droite ou ramifiée, un cycle carbocyclique ou hétérocyclique aromatique éventuellement substitué ; étant entendu qu'au moins l'un des radicaux R1' à R7' est différent d'un atome d'hydrogène ; et **R8** représente l'un des groupes de formules 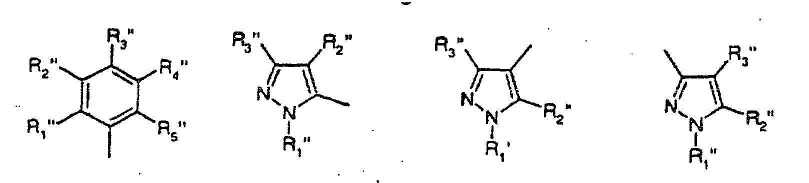 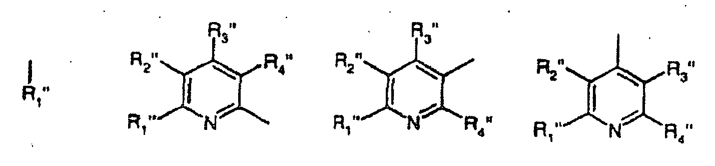 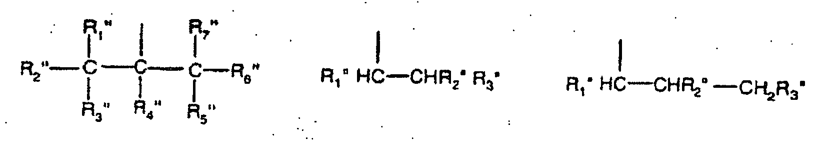 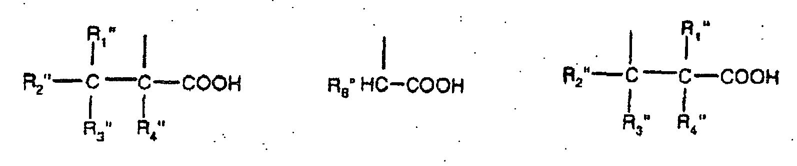 dans lesquelles **R1", R2", R3", R4", R5", R6"** et **R7"** représentent, indépendamment les uns des autres, un atome d'hydrogène, un groupe méthyle, un atome d'halogène, un groupe hydroxy, un groupe hydroxyalkyle en C₁-C₄, un groupe benzyle, un cycle carbocyclique ou hétérocyclique aromatique éventuellement substitué, un groupe méthoxy, un groupe éthoxy, un groupe carboxy, un groupe -NH₂, un groupe -NHR^{b}, un groupe -N(R^{b})₂, R^{b} représentant un atome d'hydrogène, un groupe alkyle en C₁-C₄ à chaîne droite ou ramifiée, un groupe hydroxyalkyle en C₁-C₄, un cycle carbocyclique aromatique éventuellement substitué ou un cycle hétérocyclique aromatique éventuellement substitué ;
et **R8"** est un reste requis pour la formation d'un α-aminoacide naturel.

2. Composition selon la revendication 1, **caractérisée en ce que** le composé de formules (III) à (X) est choisi parmi la 1,3,3-triméthyl-2-méthylène-indoline ainsi que ses sels, la 1,3,3,4-tétraméthyl-2-méthylène-indoline ainsi que ses sels, la 1,3,3,5-tétraméthyl-2-méthylène-indoline ainsi que ses sels, la 1,3,3,6-tétraméthyl-2-méthylène-indoline ainsi que ses sels, la 1,3,3,7-tétraméthyl-2-méthylène-indoline ainsi que ses sels, la 1,3,3,6,7-pentaméthyl-2-méthylène-indoline ainsi que ses sels, la 1,3,3,5,7-pentaméthyl-2-méthylène-indoline ainsi que ses sels, la 1,3,3,4,7-pentaméthyl-2-méthylène-indoline ainsi que ses sels, la 5-chloro-1,3,3-triméthyl-2-méthylène-indoline ainsi que ses sels, la 5-fluoro-1,3,3-triméthyl-2-méthylène-indoline ainsi que ses sels, la 5-isopropyl-1,3,3-triméthyl-2-méthylène-indoline ainsi que ses sels, la 5-hydroxy-1,3,3-triméthyl-2-méthylène-indoline ainsi que ses sels, la 5-méthoxy-1,3,3-triméthyl-2-méthylène-indoline ainsi que ses sels, la 5-amino-1,3,3-triméthyl-2-méthylène-indoline ainsi que ses sels, la 5-N-acétylamino-1,3,3-triméthyl-2-méthylène-indoline ainsi que ses sels, la 5-méthoxy-6-N-acétylamino-1,3,3-triméthyl-2-méthylène-indoline ainsi que ses sels, la 5-hydroxy-7-N-acétylamino-1,3,3-triméthyl-2-méthylène-indoline ainsi que ses sels, la 6-hydroxy-1,3,3-triméthyl-2-méthylène-indoline ainsi que ses sels, la 6-méthoxy-1,3,3-triméthyl-2-méthylène-indoline ainsi que ses sels, la 5-méthoxy-6-amino-1,3,3-triméthyl-2-méthylène-indoline ainsi que ses sels, la 5,6-dihydroxy-1,3,3-triméthyl-2-méthylène-indoline ainsi que ses sels, la 5,6-diméthoxy-1,3,3-triméthyl-2-méthylène-indoline ainsi que ses sels, la 4,5-dihydroxy-1,3,3-triméthyl-2-méthylène-indoline ainsi que ses sels, la 5,7-dihydroxy-1,3,3-triméthyl-2-méthylène-indoline ainsi que ses sels, la 5-amino-6-méthoxy-1,3,3-triméthyl-2-méthylène-indoline ainsi que ses sels, la 5-amino-7-hydroxy-1,3,3-triméthyl-2-méthylène-indoline ainsi que ses sels, la 5-hydroxy-7-amino-1,3,3-triméthyl-2-méthylène-indoline ainsi que ses sels, la 1-(2'-hydroxyéthyl)-3,3-diméthyl-2-méthylène-indoline ainsi que ses sels, le 1,3,3-triméthyl-2-méthylène-3H-benz[e]indole ainsi que ses sels et le N-éthyl-2-méthylbenzothiazole ainsi que ses sels.

3. Composition selon la revendication 1 ou 2, **caractérisée en ce que** la base de Schiff de formule (II) est choisie parmi le 4-(((2-hydroxyéthyl)imino)méthyl)-2-méthoxyphénol, le 5-(((2-hydroxyéthyl)imino)méthyl)-2-méthoxyphénol, le 2,6-diméthoxy-4-(((2-hydroxyéthyl)imino)-méthyl)-phénol, le 4-(((2-hydroxyéthyl)imino)-méthyl)phénol, le 1,2-dihydroxy-4-(((2-hydroxyéthyl)imino)méthyl)benzène, la N,N-diméthyl-4-(((2-hydroxyéthyl)imino)méthyl)aniline, le 1,2-dihydroxy-3-(((2-hydroxyéthyl)-imino)méthyl)-benzène, le 4-(((3-hydroxypropyl)imino)méthyl)-phénol, le 2,6-diméthoxy-4-(((3-hydroxypropyl)-imino)méthyl)phénol, le 4-(((2,3-dihydroxypropyl)-imino)méthylphénol, le 2,6-diméthoxy-4-(((2,3-dihydroxypropyl)imino)méthyl)phénol, le 2-[(4-hydroxy-benzylidène)-amino]-propane-1,3-diol, le 2-[(4-hydroxy-3,5-diméthoxy-benzylidène)-amino]-propane-1,3-diol, le 4-(((2-hydroxy-2-phényl-éthyl)imino)méthyl)phénol, le 2,6-diméthoxy-4-(((1-phényl-2-hydroxy-éthyl)imino)méthyl)phénol, le 4-(((2-hydroxyphényl)imino)méthyl)phénol, le 2,6-diméthoxy-4-(((2-hydroxyphényl)imino)méthyl)-phénol, l'acide 5-guanidino-2-[(4-hydroxy-benzylidène)-amino]-pentanoïque, le 2-[(4-diméthylamino-naphtalène-1-ylméthylène)-amino]-éthanol, l'acide 5-guanidino-2-[(4-hydroxy-3,5-diméthoxy-benzylidène)-amino]-pentanoïque, l'acide 2-[(4-hydroxy-3,5-diméthoxy-benzylidène)-amino]-3-(3H-imidazol-4-yl)-propionique, l'acide 2-[(4-hydroxy-benzylidène)-amino]-3-(3H-imidazol-4-yl)-propionique, l'acide 2-[(4-hydroxy-3,5-diméthoxy-benzylidène)-amino]-3-(1H-indol-3-yl)-propionique, l'acide 2-[(4-hydroxy-benzylidène)-amino]-3-(1H-indol-3-yl)-propionique, le 2-(((2-hydroxyéthyl)-imino)méthyl)phénol, le 1,2-dihydroxy-3-(((2-hydroxyéthyl)imino)méthyl)benzène, le 1,2,3-trihydroxy-4-(((2-hydroxy-éthyl)imino)méthyl)-benzène, le 1,2,3,4-tétrahydroxy-5-(((2-hydroxy-éthyl)-imino)méthyl)benzène et le 1,2,4-trihydroxy-4-(((2-hydroxyéthyl)imino)-méthyl)-benzène.

4. Composition selon l'une quelconque des revendications 1 à 3, **caractérisée en ce que** le composant (A1) a un pH de 1 à 4,5.

5. Composition selon l'une quelconque des revendications 1 à 4, **caractérisée en ce que** le composant (A2) a un pH de 7,5 à 12.

6. Composition selon l'une quelconque des revendications 1 à 5, **caractérisée en ce qu'**elle contient l'énamine de formule (III) à (X) ainsi que la base de Schiff de formule (II), par rapport à la préparation prête à l'emploi, chacun en une quantité totale de 0,01 à 10 % en poids.

7. Composition selon l'une quelconque des revendications 1 à 6, **caractérisée en ce que** les composants (A1) et (A2) se trouvent sous forme d'une solution, d'une émulsion, d'une mousse, d'une crème, d'un gel, d'une poudre, d'un produit granulé ou d'un comprimé effervescent.

8. Procédé pour la coloration temporaire de fibres, en particulier de cheveux, dans lequel la fibre est colorée avec une composition selon l'une quelconque des revendications 1 à 7 et, à un instant ultérieur quelconque, de nouveau décolorée, **caractérisé en ce que**, pour la décoloration, on laisse agir sur la fibre colorée une préparation contenant un sulfite, pendant une durée de 5 à 60 minutes, à une température de 20 à 50°C.

9. Nécessaire à plusieurs composants, constitué d'un composant (A1) contenant une énamine de formule (III) à (X) selon la revendication 1 et d'un composant (A2) contenant une base de Schiff de formule (II) selon la revendication 1.
